# EUROPEAN PATENT SPECIFICATION

(11) **EP 0 769 961 B1**
(45) Date of publication and mention of the grant of the patent: **02.11.2006**
(21) Application number: 95926645.3
(22) Date of filing: 07.07.1995
(51) Int. Cl.: A61K 38/51, A61P 17/02

(54) **USE OF BACTERIAL ENZYMES E.G. HEPARINASES, CHONDROITINASES FOR MODULATING WOUND HEALING PROCESSES**
VERWENDUNG VON BAKTERIELLEN ENZYMEN Z.B. HEPARINASEN, CHODROITINASEN UM WUNDHEILUNGPROZESSE ZU MODULIEREN
UTILISATION D'ENZYMES BACTERIEL P.EX. HEPARINASES, CHONDROITINASES POUR MODULER DES PROCESSUS DE CICATRISATION

(30) Priority: 08.07.1994 US 273109
(43) Date of publication of application: 02.05.1997
(73) Proprietor: Biomarin Pharmaceutical Inc., Novato CA 94949 (US)
(72) Inventor: ZIMMERMANN, Joseph, Elm Grove, WI 53122 (US); VLODAVSKY, Israel, 93845 Jerusalem (IL); BENNETT, D., Clark, Pierrefonds, Quebec H4Y 1Z4 (CA); DANAGHER, Pamela, Montreal, Quebec H3V 2G4 (CA); BROUGHTON, Richard, Montreal, Quebec H3V 1C2 (CA)
(74) Representative: Bassett, Richard Simon
(86) International application number: PCT/US1995/008608
(87) International publication number: WO 1996/001648

(56) References cited:
- WO-A-91/02977
- WO-A-95/13091
- WO-A-95/13830
- PROCEEDINGS OF THE NATIONAL ACADEMY OF SCIENCES OF USA, vol. 91, no. 4, 15 February 1994 WASHINGTON US, pages 1524-1528, SASISEKHARAN R. ET AL. 'Heparinase inhibits neovascularization'
- JOURNAL OF BIOLOGICAL CHEMISTRY, vol. 267, no. 34, 5 December 1992 MD US, pages 24347-24355, LOHSE D.L. ET AL. 'Purification and Characterization of Heparin Lyases from Flavobacterium heparinum' cited in the application
- PROCEEDINGS OF THE NATIONAL ACADEMY OF SCIENCES OF USA, vol. 90, no. 8, 15 April 1993 WASHINGTON US, pages 3660-3664, SASISEKHARAN R. ET AL. 'Cloning and expression of heparinase I gene from Flavobacterium heparinum' cited in the application

## Description

The present invention describes the use of bacterial glycosaminoglycan degrading enzymes to modulate events in the wound healing process.

Growth factors are naturally occurring polypeptides that elicit hormone type modulation of cell proliferation and differentiation. The mechanism by which these events transpire is typically initiated by the growth factor contacting specific receptors or receptor systems which are located on the cell surface. The sequence of intracellular events that occur subsequent to the receptor/growth factor interaction are responsible for mitogenic and differentiating responses by the cell. These mechanisms are not fully understood but may include activation of tyrosine kinases, nucleotide metabolism and variations in cell electrolyte levels (Burgess and Macaig, Ann. *Rev. Biochem,* 58:575-606, 1989).

For most cell types, events of mitogenesis and differentiation are subdued in the normal adult animal. These growth factor mediated events are more commonly associated with developing organisms, during wound healing processes or in various disease states including cancer and vascular disease. For example, the normal turnover rate of endothelial cells, including the lining of microvessels and arteries, is measured in thousands of days. During normal wound healing however, these endothelial cells proliferate rapidly, with a turnover rate of approximately five days (Folkman and Shing, *J. Biol. Chem.* 267(16):10931-10934, 1992). The increase in proliferation that occurs during wound healing appears to be the result of an increase in the local concentration of various angiogenic molecules, including growth factors.

The fibroblast growth factor family includes at least seven polypeptides that have been shown to stimulate proliferation in various cell lines including endothelial cells, fibroblasts, smooth muscle cells and epidermal cells. Included in this group are acidic fibroblast growth factor (FGF-1), basic fibroblast growth factor (FGF-2), int-2 (FGF-3), Kaposi sarcoma growth factor (FGF-4), hst-1 (FGF-5), hst-2 (FGF-6) and keratinocyte growth factor; (FGF-7) (Baird and Klagsbrun, Ann. *N.Y. Acad. Sci.* 638:xiv, 1991). These molecules, and other cytokines including tissue growth factors, TGFα and TGFβ, platelet derived growth factors, PDGF, granulocyte-macrophage colony stimulating factor, GM-CSF, interleukin 3, IL-3, and platelet factor 4, PF4, share a common feature in their affinity for heparin (Clark, *Dermatol. Clin.* 11:647-666, 1993). Specific cell type responses also have been associated with particular factors. EGF and TGFα stimulate the proliferation of keratinocytes, TGFβ stimulates collagen and fibronectin synthesis, PDGF stimulates angiogenesis and granulation tissue formation and FGF-7 stimulates epithelial cell proliferation (Staiano-Coico, et al., *J. Exp. Med.* 178:865-878, 1993). PDGF, FGF-2 and a recently described heparin binding epidermal growth factor HB-EGF (Higashiyama, et. al., *Science* 251:936-939, 1991) additionally are involved in the proliferation and migration of vascular smooth muscle cells and vascular endothelial cells.

The change in a cell's metabolic state from quiescent to proliferative or migratory implies an enhanced availability of the appropriate signaling molecules in the vicinity of the cell. In principle this could result from either an increase in growth factor synthesis or the release of growth factors from storage reservoirs. In nature, both mechanisms have been observed. The expression of FGF-1, FGF-2, FGF-5 and FGF-7 are upregulated after full thickness dermal injury (Werner, et. al., *Proc. Natl. Acad. Sci.* 89:6896) while TGFβ, FGF-2 and PDGF synthesis increases in smooth muscle cells in response to vascular injury. Growth factors also have been detected in most solid tissues extracted from normal adult, non-wounded samples. Despite the presence of growth factors in these areas, the cells comprising them are not in a proliferative state. Apparently, growth factors are stored outside the cell in basement membranes and the extracellular matrix where they are prevented from contacting their respective cell surface receptors. In this mode they serve as an emergency supply for wound repair and blood vessel formation functions (Vlodavsky, et. al. *TIBS* 16:268-271, 1991).

An initial event in tissue or vessel injury may involve a mechanical dislodging of growth factors from the extracellular space, making them available to cell surface receptors where they stimulate cell proliferation and cell synthesis of additional growth factors. Alternately, cells under stress may secrete molecules which displace the extracellular growth factors from these storage reservoirs. Tumor cells have been shown to secrete degradative enzymes, including proteoglycanases, collagenases and metalloproteinases, coincident with metastasis (Nicolson *Curr. Opinion Cell Biol.* 1:1009-1019, 1989). In addition to facilitating tumor migration through blood vessels, the destruction of extracellular matrix components releases growth factors, thereby promoting new blood vessel formation which feeds the growing tumor mass (Folkman, et al., *Am J Pathol* 130:393-400, 1988).

Extracellular matrices (ECM) are multi-component structures synthesized by and surrounding various cell types including endothelial, epithelial, epidermal and muscle cells. The ECM is formed largely of collagen and heparan sulfate proteoglycans. It also contains fibronectin, chondroitin sulfate proteoglycans and smaller proteins. Growth factors are sequestered in these matrices by association with the glycosaminoglycan portion of the heparan sulfate proteoglycans. Heparin and heparan sulfate are polysaccharides formed of alternating hexuronic, either D-glucuronic or L-iduronic, and glucosamine, N-acetylated or N-sulfated, residues with varying sulfation patterns. Heparin extracted from porcine intestines, bovine lungs or human mast cells displays a high degree of sulfation, up to 2.6 sulfates per disaccharide unit, and a greater iduronic acid content than heparan sulfate. Conversely, heparan sulfate has a lower degree of sulfation and preferentially contains glucuronic acid in the alternating saccharide position. "Heparin like" regions of high iduronic acid and high sulfation have been associated with the bFGF binding region of heparan sulfate from human fibroblasts (Turnbull, et al., *J. Biol. Chem.* 267(15) 10337-10341, 1992). However, the composition of heparan sulfate in the extracellular matrix has not been fully characterized.

The stimulation of cell proliferation and migration by growth factors constitutes one of the events in the wound healing process which is a multifactoral interactive process involving biochemical mediators, the extracellular matrix and parenchymal cells. The wound healing process is generally divided into three temporally overlapping phases: inflammation, proliferation and remodeling. During inflammation, blood borne cells infiltrate the wound site and release several mediating molecules including platelet derived growth factor, von Willibrand factor, thrombospondin, fibronectin, fibrinogen, 5-hydroxytryptophan, thromboxane-A2 and adenosine diphosphate (Kirsner and Eaglstein, *J*. *Dermatol.* 151:629-640, 1993). A platelet plug and thrombus are formed and provide a matrix for monocytes, fibroblasts and keratinocytes. Chemotactic molecules attract monocytes which transform into macrophages and secrete additional growth factors (Nathan and Sporn, *J. Cell Biol.* 113:981-986, 1991). Neutrophils may assist in this process by secreting the degradative enzymes elastase and collagenase which enhance the passage of cells through the basement membranes.

Keratinocytes and epidermal cells, which are involved in the closure of dermal wounds, migrate to the wound site during the proliferative phase. Angiogenesis, the formation of new blood vessels in response to chemoattractant and angiogenic signals (Folkman and Klagsbrun, *Science* 235:442-447, 1987), and fibroplasia, the accumulation of fibroblasts and formation of granulation tissue, also occurs during the proliferative phase. Tissue remodeling is accompanied by the secretion of matrix components, including fibronectin, collagen and proteoglycans which serve as a scaffold for cellular migration and tissue support. Type III collagen, synthesized in the earlier stages of wound healing, is replaced by the more permanent type I form through a process of proteolytic turnover.

Ischemia refers to the pathological condition due to the localized dysfunction of the vascular system resulting in inadequate blood supply with subsequent tissue damage. In this case revascularization, whether through the stimulation of angiogenesis or by surgical methods, must precede the normal wound healing course of the damaged tissue.

The action of enzymes which degrade components of the extracellular matrix and basement membranes may facilitate the events of tissue repair by a variety of mechanisms including the release of bound cytokines entrapped by heparan sulfate and by increasing the permeability of the matrix, thereby enhancing the mobility of mediator molecules, growth factors and chemotactic agents, as well as the cells involved in the healing process. Glycosaminoglycans are subject to degradation by a variety of eukaryotic and prokaryotic enzymes. Heparan sulfate degrading activity has been detected in platelets (Oldberg et al. *Biochemistry,* 19:5755-5762, 1980), tumor cells (Nakajima, et al. *J. Biol. Chem.* 259:2283-2290, 1984) and endothelial cells (Gaal et al. *Biochem. Biophys. Res. Comm.,* 161:604-614, 1989). These heparanase enzymes act by catalyzing the hydrolysis of the carbohydrate backbone of heparan sulfate at the hexuronic acid (1 -> 4) glucosamine linkage (Nakajima et al., *J. Cell, Biochem.,* 36:157-167, 1988). Mammalian heparanases are typically inhibited by the highly sulfated heparin form of the heparin-heparan sulfate family. However, accurate biochemical characterizations of these enzymes have thus far been prevented by the lack of a method to obtain homogeneous preparations of the molecules.

Heparin degrading enzymes also have been found in microorganisms including *Flavobacterium heparinum* (Lohse and Linhardt, *J. Biol. Chem.* 267:2437-24355, 1992), *Bacteroides* strains (Saylers, et al., *Appl. Environ. Microbiol.* 33:319-322, 1977; Nakamura, et al., *J. Clin. Microbiol.* 26:1070-1071, 1988), *Flavobacterium* Hp206 (Yoshida, et al., 10th Annual Symposium of Glycoconjugates, Jerusalem 1989) and *Cytophagia* species (Bohn, et al., *Drug Res.* 41(I), Nr. 4:456-460, 1991). Chrondoitin sulfate degrading enzymes have been isolated from several microorganisms including *Flavobacterium heparinum* (Michaleacci, et al., *Biochem. J*. 151:123, 1975), *Bacteroides* species (Saylers, et al. *J. Bacteriol.* 143:781, 1980; Linn, et al., *J. Bacteriol.* 156:859, 1983; Steffen, et al., *J. Clin. Microbiol.* 14:153, 1981), *Proteus vulgaris* (Uamagata, et al., *J. Biol. Chem.* 243:1523, 1968, Suzuki, *Meth. Enzymol.* 28:911, 1972), Beneckea, *Microcossus* and *Vibrio* species (Kitamikada and Lee, *Appl. Microbiol.* 29:414, 1975) and *Arthrobacter aurescens* (Hiyam and Okada, *J*. *Biol. Chem.* 250:1824-1828, 1975).

*F. heparinum* produces three forms of heparinase, heparinase 1, heparinase 2, and heparinase 3 (heparitinase) (Lohse and Linhardt, *J*. *Biol. Chem.* 267:24347-24355, 1992). All three enzymes cleave at glucosamine (1 -> 4) hexuronic acid linkages with differing degrees of specificity depending on sulfation patterns and particular hexuronic acid residue, iduronic or glucuronic, in a particular cleavage site (Desai, et al., *Arch. Biochem. Biophys.* 306:461-468, 1993). *F. heparinum* also produces two enzymes which degrade members of the chondroitin sulfate/dermatan sulfate family. These are chondroitin lyase AC, which degrades both chondroitin sulfate A and chondroitin sulfate C by cleaving the galactosamine (1 -> 4) glucuronic acid linkage in the polysaccharide backbone and chondroitin lyase B which degrades dermatan sulfate (chondroitin sulfate B) by cleaving the galactosamine (1 -> 4) iduronic acid linkage in the polysaccharide backbone. The enzymatic mechanism of the *F. heparinum* enzymes is through an elimination reaction, thereby differentiating them from the mammalian glycosaminoglycan degrading enzymes. Furthermore, none of the *F. heparinum* lyase enzymes appear inhibited by glycosaminoglycan molecules as are the mammalian enzymes.

Mammalian heparanase, partially purified from tumor cell line extracts, as well as heparinase 1 and heparinase 3 from *Flavobacterium heparinum,* have been shown to release ¹²⁵I radiolabelled FGF-2 that had been pre-adsorbed to extracellular matrix synthesized in vitro by bovine aorta endothelial cells (Bashkin, et al. *J. Cell.* Physiol. 167:126-137, 1992). However, since unfractionated and low molecular weight heparin elicited a similar release of the exogenously absorbed ¹²⁵I radiolabelled FGF-2, it is not clear from these reports whether the measured release was due to the enzymatic degradation of the heparan sulfate in the ECM or an ion exchange type electrolytic displacement of FGF-2 from the negatively charged heparan sulfate. The same research group reported the release of growth promoting activity from vascular smooth muscle cells by treatment with heparinase 3 and from extracellular matrix by exposure to extracts of neutrophils or lymphoma cells. However, there has been no demonstration of the release of growth promoting activity from extracellular matrix by contact with bacterial glycosaminoglycan degrading enzymes nor have these enzymes been shown to promote tissue repair or new vessel growth *in vivo.*

### Summary of the Invention

Glycosaminoglycans, including heparinases 1, 2 and 3 as well as chondroitinases AC and B from the Gram negative bacterium *Flavobacterium heparinum,* can be used either separately or in combination to manipulate cell proliferation. In one embodiment, heparinases are administered to degrade heparan sulfate components of the extracellular matrix, thereby allowing the heparin binding growth factors which are stored in the extracellular matrix to migrate to adjacent cells. The mobility of chemoattractant agents, growth factors and cells can also be increased by treating tissues with glycosaminoglycan degrading enzymes, both chondroitinases and heparinases. The enzymatic removal of chondroitin sulfates from cell surfaces effectively increases the availability of growth factor receptors on the cell's surface. Selectively removing heparan sulfate from cell surfaces while leaving the extracellular matrix intact, conversely inhibits cell proliferation by down regulating the cell's response to growth factors. This is achieved by targeting heparin or heparan sulfate degrading activities to the cell surface. Targeting the heparin degrading activity can be achieved by genetically engineering a ligand binding functionality into the heparinase proteins, or by physically controlling the localized enzyme concentration through the method of administration.

Methods for preparing glycosaminoglycan enzymes and genetically engineered derivatives of them as well as methods for producing pharmaceutical preparations of highly purified glycosaminoglycan degrading enzymes are described. Methods are disclosed to produce derivatives of the heparin degrading enzymes which incorporate binding properties of other proteins. These molecules can be used to target the heparin degrading activity to the cell surface which inhibit a cell's response to endogenous growth factors.

Examples demonstrate the release of growth promoting activity from ECM and intact cells and tissues using glycosaminoglycanases, enhancement of cell proliferation in vitro by treatment with glycosaminoglycanases, especially heparinase 3, the growth promoting activity of heparan sulfate fragments released by cells treated with glycosaminoglycanases and the effectiveness of heparinase 3 in stimulating wound healing *in vivo* in animal models.

Accordingly the invention provides use of a pharmaceutical composition in the manufacture of a medicament for enhaning wound healing by release of heparin binding growth factors and molecules from the extracellular matrix, removal of chondroitin sulfate from cell surface receptors and/or removal of the heparan sulfate component of their growth factor receptor complex wherein the composition comprises a bacterial glycosaminoglycan degrading enzyme selected from the group consisting of heparinase 1 from *Flovobacterium heparinum,* heparinase 2 from *Flovobacterium heparinum,* heparinase 3 from *Flavobacterium heparinum,* chondroitinase AC from *Flovobacterium heparinum,* and chondroitinase B from *Flavobacterium heparinum,* heparinase from *Bacteroides* strains, heparinase from *Flavobacterium* Hp206, heparinase from *Cytophagia* species, chondroitin sulfate degrading enzymes from *Bacteroides* species, chondroitin sulfate degrading enzymes from *Proteus vulgaris,* chondroitin sulfate degrading enzymes from *Microcossus,* chondroitin sulfate degrading enzymes from *Vibrio* species, chondroitin sulfate degrading enzymes from *Arthrobacter aurescens,* these enzymes expressed from recombinant nucleotide sequences expressed in bacteria, and combinations thereof, in combination with a pharmaceutically acceptable carrier for localized administration.

The invention also provides :
a delivery vehicle for delivery of, and comprising, enzyme in combination with the carrier in a dosage effective to enhance wound healing by release of heparin binding growth factors and molecules from the extracellular matrix, removal of chondroitin sulphate from cell surface receptors and/or removal of the heparan sulphate component of their growth factor receptor complex,
   wherein the enzyme is a bacterial glycosaminoglycan degrading enzyme selected from the group consisting of heparinase 1 from *Flavobacterium heparinum,* heparinase 2 from *Flovobacterium heparinum,* heparinase 3 from *Flavobacterium heparinum,* chondroitinase AC from *Flavobacterium heparinum,* and chondroitinase B from *Flavobacterium heparinum,* heparinase from *Bacteroides* strains, heparinase from *Flavobacterium* Hp206, heparinase from *Cylophagia* species, chondroitin sulfate degrading enzymes from *Bacteroides* species, chondroitin sulfate degrading enzymes from *Proteus vulgaris,* chondroitin sulfate degrading enzymes from *Microcossus,* chondroitin sulfate degrading enzymes from *Vibrio* species, chondroitin sulfate degrading enzymes from *Arthrobacter aurescens,* these enzymes expressed from recombinant nucleotide sequences expressed in bacteria, and combinations thereof, in combination with a pharmaceutically acceptable carrier wherein, if the enzyme is heparinase 1, 2 or 3 from Flavobacterium heparinum, the composition does not comprise an effective amount of heparinase in a pharmaceutically acceptable carrier to inhibit angiogenesis at a selected site in a non-heparinised patient.

- the use of a delivery vehicle of the invention in the manufacture of a device for enhancing wound healing by release of heparin binding growth factors and molecules from the extracellular matrix, removal of chondroitin sulphate from cell surface receptors and/or removal of the heparan sulphate component of their growth factor receptor complex;
- a composition comprising a bacterial glycosaminoglycan degrading enzyme as described above wherein, if the enzyme is heparinase 1, 2 or 3 from Flavobacterium heparinum, the composition does not comprise an effective amount of heparinase in a pharmaceutically acceptable carrier to inhibit angiogenesis at a selected site in a non-heparinised patient.
- a composition comprising a bacterial glycosaminoglycan degrading enzyme as described above wherein the carrier is selected from the group consisting of polymeric films, microparticulates, microcapsules, protesomes, liposomes, transdermal patches and bandages.

### Brief Description of the Drawings

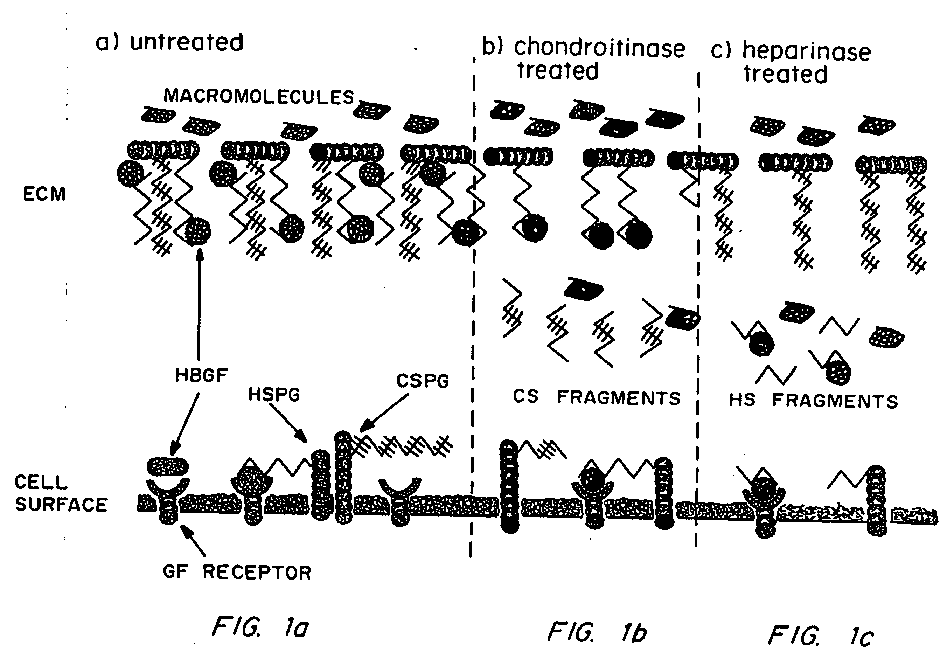

Figures 1a, 1b and 1c are schematic diagrams depicting the function of glycosaminoglycans in the extracellular matrix (ECM - top half) and on cell surfaces (bottom half).
Figure la shows that the heparan sulfate component (plain squiggled line) of heparan sulfate proteoglycans (HSPG) binds to heparin binding growth factors (HBGF) in both the extracellular matrix and at the cell surface. Growth factors not bound to heparan sulfate are unable to bind their cell surface receptor. Heparan sulfate or fragments of heparan sulfate attach to the growth factors and elicit a conformational change which allows binding to the receptor. Chondroitin sulfate (hatched squiggled line) proteoglycans (CSPG) also are located in the extracellular matrix and on the cell surface. At the cell surface the chondroitin sulfate molecules may sterically hinder the access of heparin binding growth factor receptors. Figure 1b shows that treatment with chondroitin sulfate degrading enzymes allows greater access to the cell surface receptors and increases the mobility of molecules such as chemoattractants, growth factors and cells through the extracellular matrix. Figure 1c shows that treatment with heparin or heparan sulfate degrading enzymes releases heparan sulfate fragments and heparin binding growth factors from the extracellular matrix, thereby increasing their availability to the adjacent cell surface receptors, and increases the mobility of molecules such as chemoattractants, growth factors and cells through the extracellular matrix.

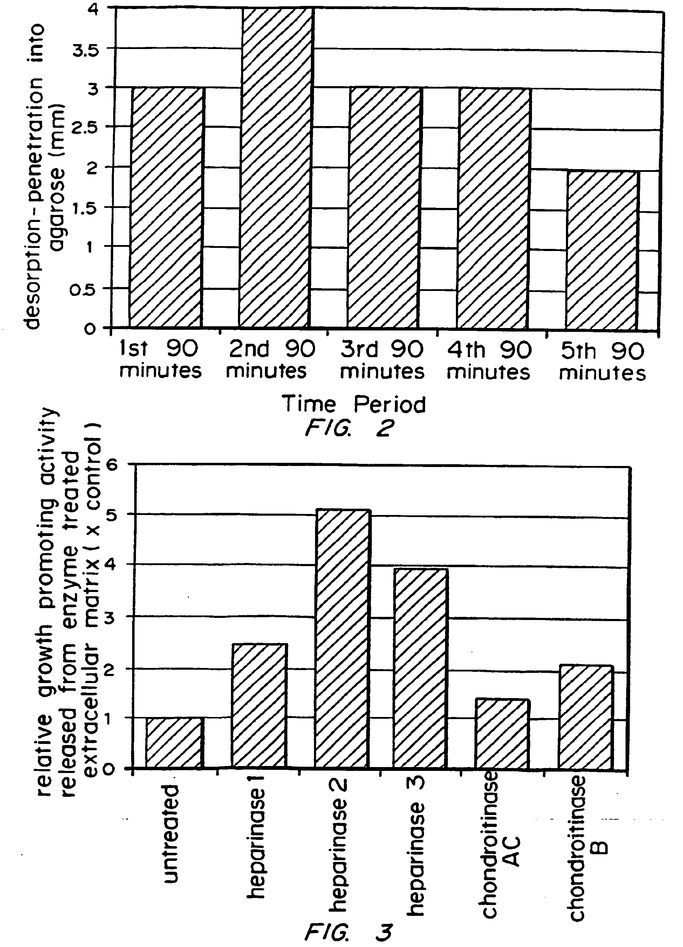

Figure 2 is a graph of the desorption (penetration into agarose (mm) over time (minutes)) of heparinase into semi-solid gels to measure the amount of enzyme present.

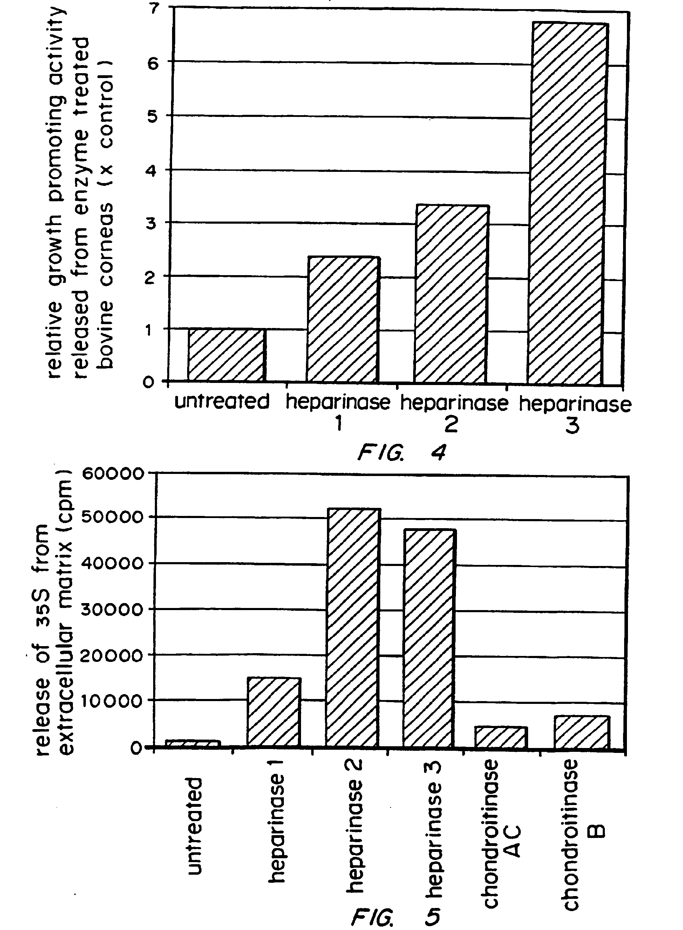

Figure 3 is a graph of relative growth promoting activity released from enzyme treated extracellular matrix (x control) for untreated, heparinase 1, heparinase 2, heparinase 3, chondroitinase AC and chondroitinase B. The results are expressed as the ratio of thymidine incorporation by Balb/c 3T3 fibroblasts exposed to enzyme treated matrix supernatants to that of untreated matrix supernatants.
Figure 4 is a graph of the relative growth promoting activity released from enzyme treated bovine corneas (x control) for untreated, heparinase 1, heparinase 2, and heparinase 3. The results are expressed as the ratio of thymidine incorporation by Balb/c 3T3 fibroblasts exposed to enzyme treated corneal supernatants to that of untreated corneal supernatants.

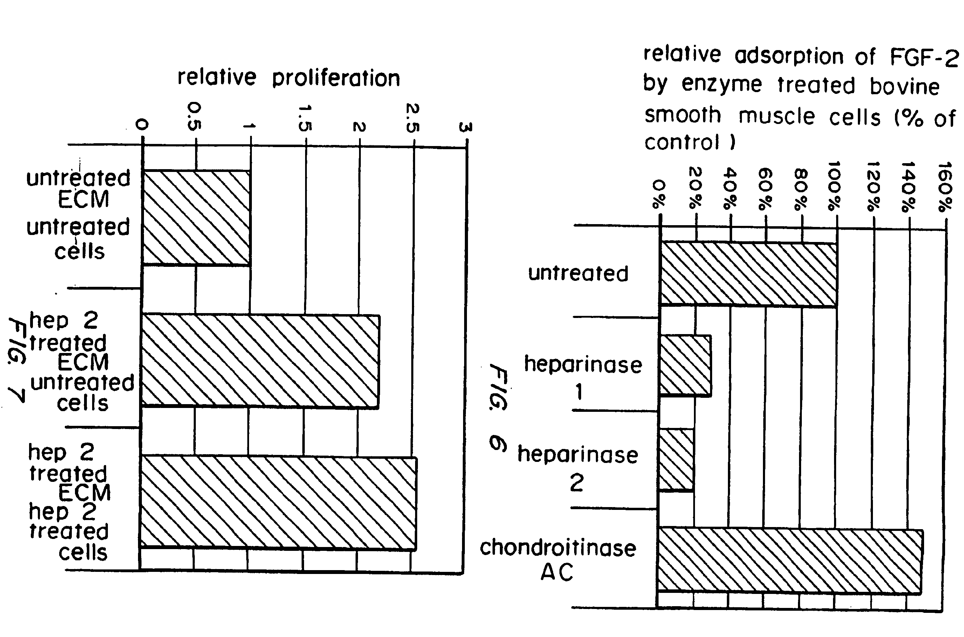

Figure 5 is a graph of the release of ³⁵S from extracellular matrix (cpm) for untreated, heparinase 1, heparinase 2, heparinase 3, chondroitinase AC, and chondroitinase B.
Figure 6 is a graph of the relative absorption of FGF-2 by enzyme treated bovine smooth muscle cells (% of control) for untreated, heparinase 1, heparinase 2, and chondroitinase AC.
Figure 7 is a graph of the relative proliferative response of Balb/C 3T3 fibroblasts to enzymatic treatment of either the cell surface, extracellular matrix or both. Proliferation was determined by ³H-thymidine incorporation and is expressed as the ratio of incorporation observed in the treated conditions to that of the control (untreated cells exposed to supernatant from untreated matrix).

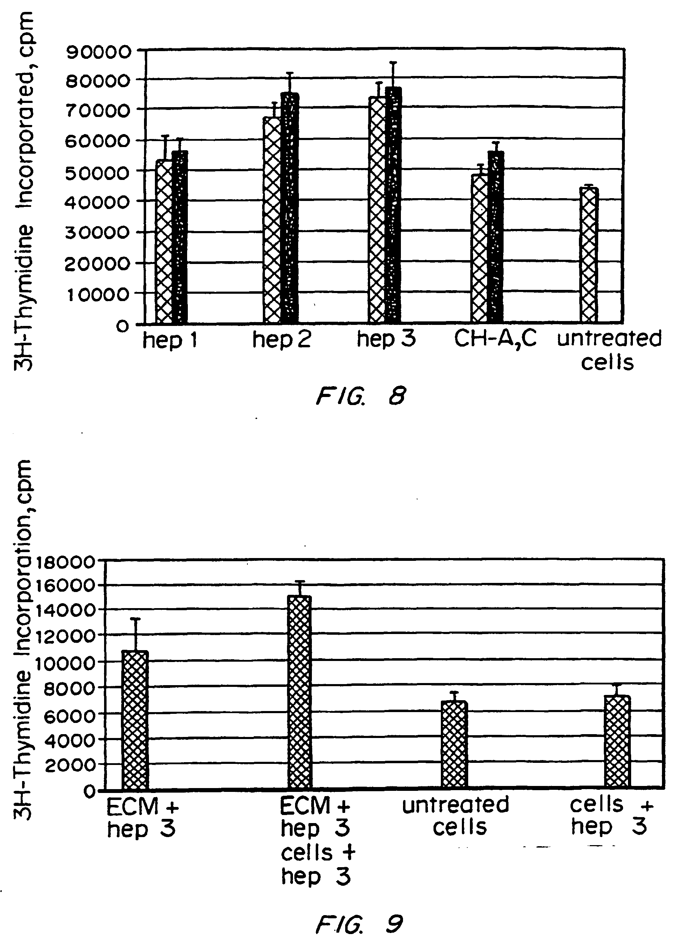

Figure 8 is a graph of the proliferative response of quiescent balb/C 3T3 fibroblasts to soluble material released from enzyme treated extracellular matrix for heparinase 1, 2, 3, and chondroitinase AC (hatched bars). The proliferative response of cells treated with heparinase 1, 2, 3, or chondroitinase AC prior to their exposure to enzyme treated matrix supernatants is also shown (solid bars). These results are expressed as the counts per minute of ³H-thymidine incorporated by balb/C 3T3 fibroblasts exposed to enzyme treated matrix supernatants. The negative control "untreated cells" represents the proliferative response of cells exposed to supernatant from untreated matrix.
Figure 9 is a graph of the proliferative response of quiescent balb/C 3T3 fibroblasts, both pretreated with heparinase 3 and untreated, to soluble material released from enzyme treated extracellular matrix for heparinase 3. The proliferative response of quiescent balb/C 3T3 fibroblasts, both pretreated with heparinase 3 and untreated, to material released spontaneously from extracellular matrix, is presented. These results are expressed as the counts per minute of ³H-thymidine incorporated by balb/C 3T3 fibroblasts exposed to enzyme treated matrix supernatants. Untreated cells are cells exposed to supernatant from untreated matrix; cells + hep 3 are cells pretreated with heparinase 3 prior to exposure to supernatant from untreated matrix; ECM + hep 3 are cells exposed to supernatant from heparinase 3 treated matrix; and ECM + hep 3 cells + hep 3 are heparinase 3 treated cells exposed to supernatant from heparinase 3 treated matrix.

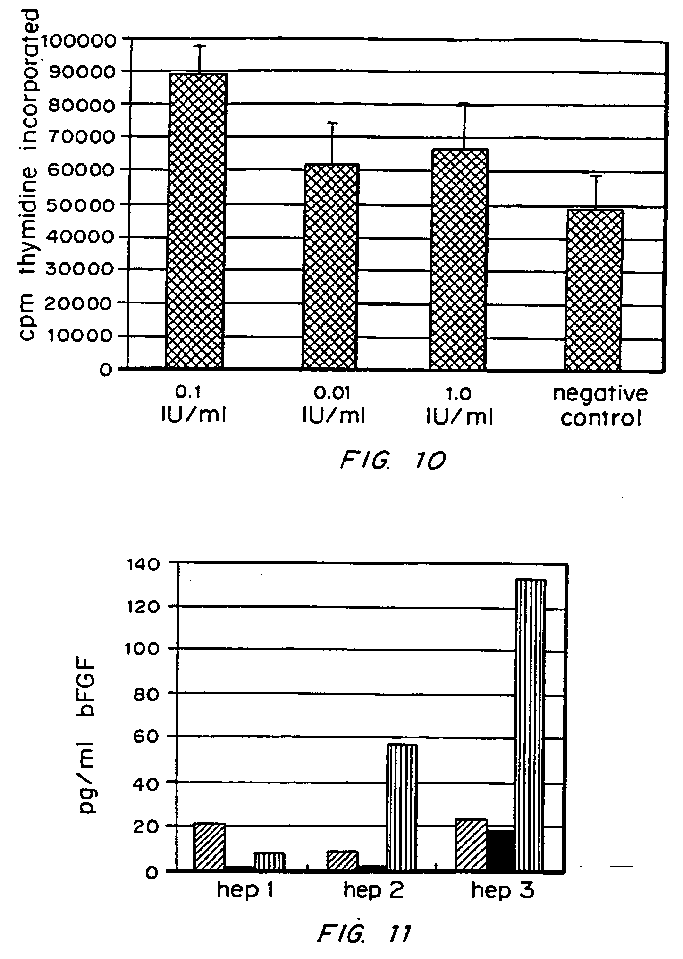

Figure 10 is a graph of the proliferative response of quiescent balb/C 3T3 fibroblasts to soluble material released from enzyme treated bovine corneas for three concentrations of heparinase 3, 0.1, 0.01, and 1.0 IU/ml. The negative control value "untreated cells" represents the proliferative response of cells exposed to supernatant from untreated corneas.

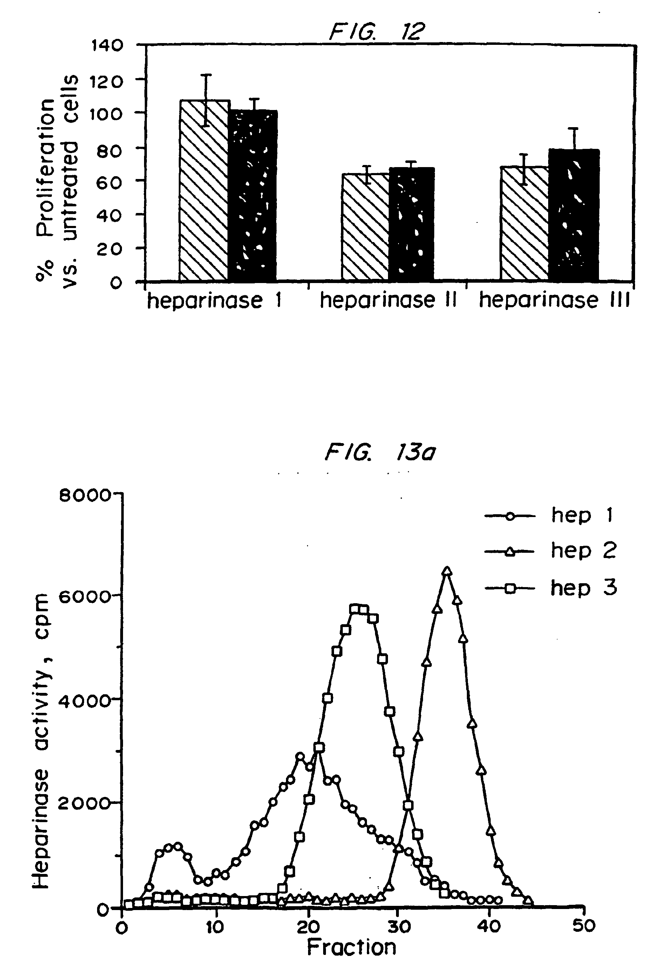

Figure 11 is a graph depicting the release of bFGF from bovine endothelial cells (hatched bars), bovine smooth muscle cells (solid bars), and extracellular matrix (gray bars) exposed to heparinase 1, 2, or 3.
Figure 12 is a graph of the inhibition of smooth muscle cell proliferation by treatment of the cells with heparinase 1, heparinase 2, and heparinase 3. The results are expressed as the percent of cell proliferation after enzyme treatment as compared to untreated control cells. Untreated cells have a proliferative level of 100%. The bars with diagonal lines are from treatments with 0. 1 IU/ml of enzyme. The solid bars are from treatments with 0.5 IU/ml of enzyme.
Figure. 13A is a graph of the degradation of sulfate labeled ECM by heparinase 1, 2 or 3. Metabolically sulfate labeled ECM coating 4-well tissue culture plates was incubated for 18 h at 37°C with 0.1 U/ml heparinase 1 (o), heparinase 2 (△) or heparinase 3 (□). Sulfate labeled material released into the incubation medium was analyzed by gel filtration on Sepharose 6B.

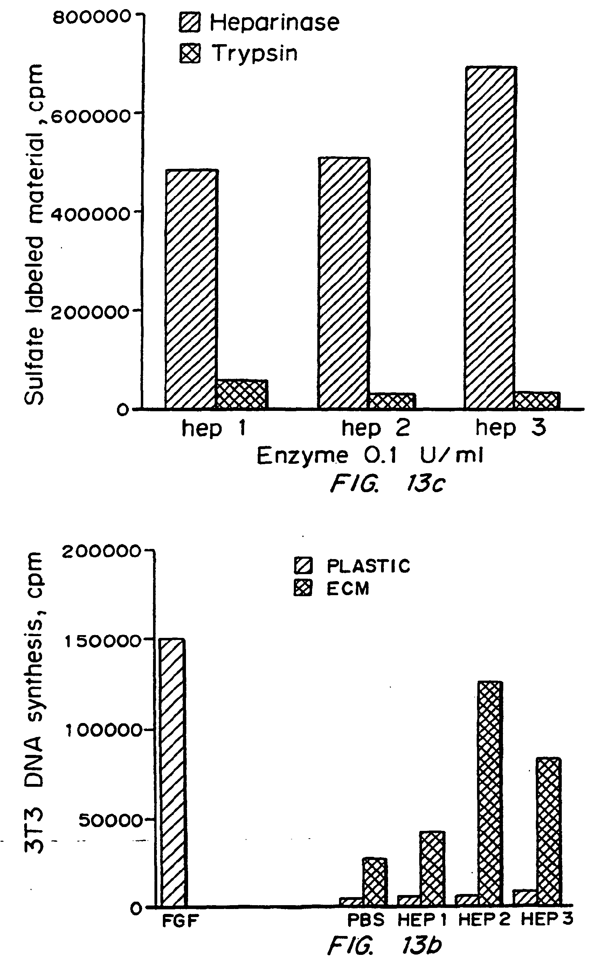

Figure 13B is a graph of the release of ECM-bound mitogenic activity. Aliquots of the incubation medium on plastic (diagonal line shading) or ECM (cross-hatched shading) were tested for stimulation of ³H-thymidine incorporation in growth arrested 3T3 fibroblasts.
Figure 13C is a graph of degradation of sulfate labeled labeled ECM by heparinase 1, 2 and 3. Sulfate labeled ECM was incubated for 18 h at 37°C with 0.1 U/ml heparinase 1, 2 or 3. The total amount of sulfate labeled-material released into the incubation medium (diagonal line shading) was counted. The remaining ECM was digested for one h at 37°C) with trypsin (5 µg/ml) and the solubilized radioactivity (cross-hatched shading) counted in a β-scintillation counter.

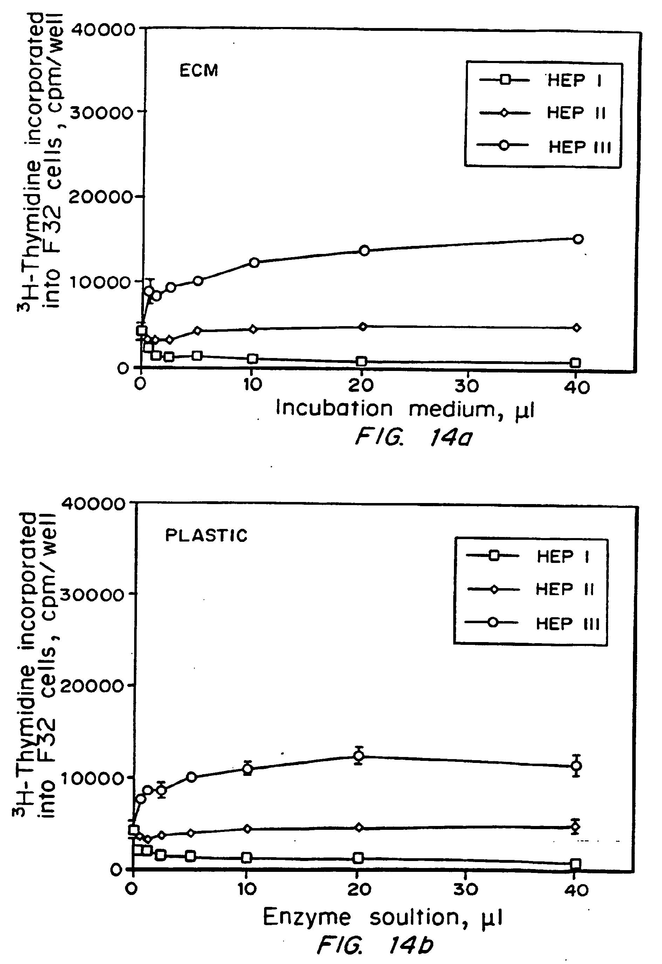

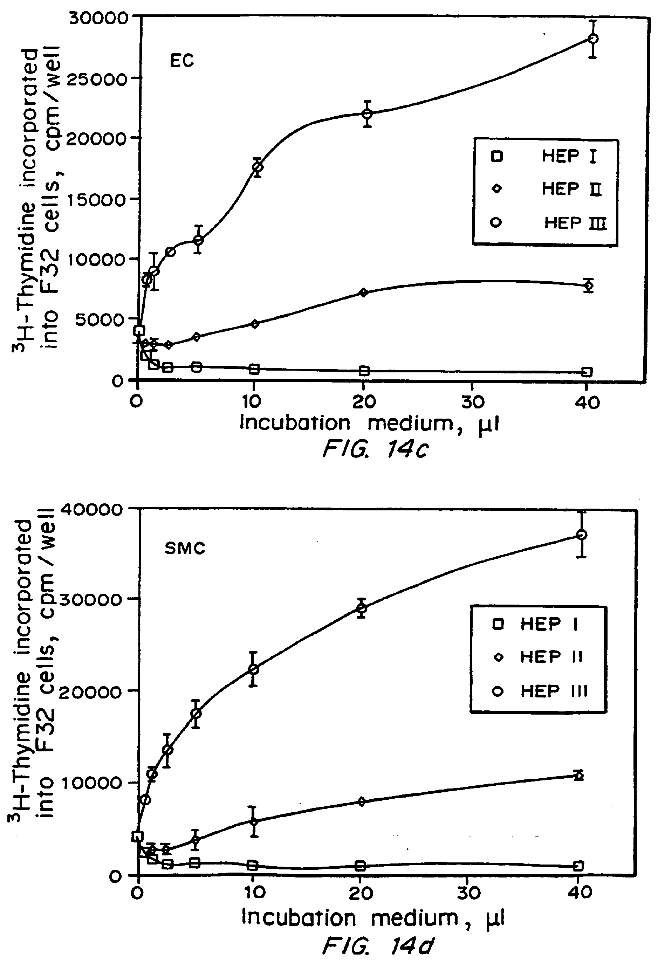

Figures 14A,14B, 14C and 14D are graphs of stimulation of F32 lymphoid cell proliferation by heparan sulfate fragments released from cells and ECM exposed to heparinase 1, 2 or 3 on different surface. Regular (plastic) (Figure 14B) and ECM coated (Figure 14A) 4 well plates as well as confluent cultures of vascular endothelial cells (EC) (Figure 14C) and smooth muscle cells (SMC) (Figure 14D) were incubated for 1 h at 37°C with 0.1 U/ml heparinase 1 (□), heparinase 2 (◇) or heparinase 3 (○) . Aliquots (1-40 µl) of the incubation media were then added to F32 cells seeded into 96 well plates in the presence of 5 ng/ml bFGF. ³H-thymidine (1 µCᵢ/well) was added 48 h after seeding and 6 h later the cells were harvested and measured for ³H-thymidine incorporation. Each data point represents the mean ±S.D. of six culture wells.

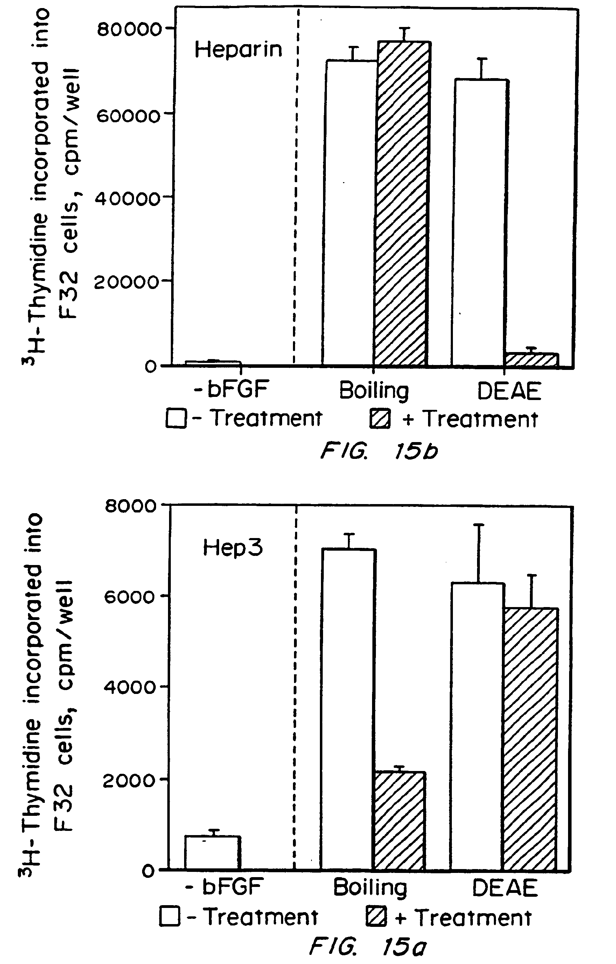

Figures 15A and 15B are graphs of the stimulation of F32 lymphoid cell proliferation by Heparinase 3. Figure 15A is a graph of incubation of F32 lymphoid cells in 96 well plates with 5 ng/ml bFGF in the absence and presence of 0.1 U/ml native (no shading) or heat inactivated (10 min, 95°C) (diagonal line shading) heparinase 3. The heparinase 3 enzyme (0.1 U/ml) was also applied onto DEAE cellulose (0.5 ml) and both the loading (no shading) and flow through (diagonal line shading) material were added to the F32 lymphoid cells. Figure 15B is a graph of incubation of F-32 lymphoid cells in 96 well plates with 5 ng/ml bFGF in the absence and presence of 1 µg/ml native (no shading) or heat inactivated (10 min, 95°C) (diagonal line shading) heparin. The heparin was also applied onto DEAE cellulose (0.5 ml) and both the loading (no shading) and flow through (diagonal line shading) material was added to the F32 lymphoid cells. ³H-thymidine (1 µCᵢ/well) was added to each well 48 h after seeding and 6 h later the cells were harvested and measured for ³H-thymidine incorporation. Each data point represents the mean ± S.D. of six culture wells.

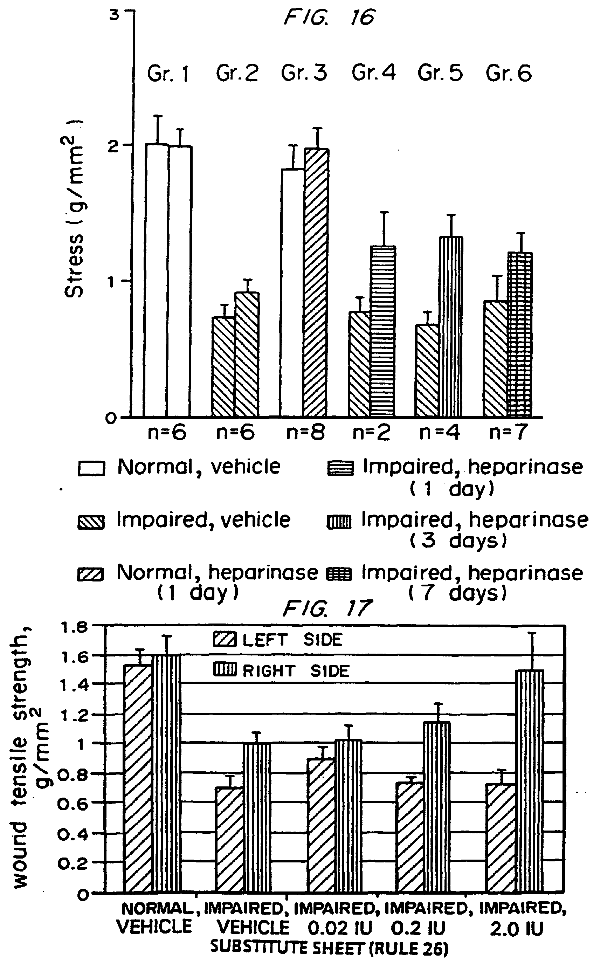

Figure 16 is a graph of the strength (tension, g/mm²) of skin containing a wound site taken from six groups of rats following *in vivo* testing of the ability of heparinase to stimulate wound healing, comparing the effect of vehicle, heparinase (1 day), and impaired (heat inactivated) heparinase at 1, 3 and 7 days.
Figure 17 is a graph of the strength (tension, g/mm²) of skin containing a wound site taken from six groups of rats following *in vivo* testing of heparinase in dosages of 0.02, 0.2, and 2.0 IU heparinase 3 to stimulate wound healing.

### Detailed Description of the Invention

A methodology for controlling events involved in wound healing processes by the use of highly purified glycosaminoglycan degrading enzymes derived from *Flavobacterium heparinum* genes is disclosed. Glycosaminoglycans, including heparan sulfate, chondroitin sulfate and dermatan sulfate, are the sulfated polysaccharide components of proteoglycans located on cell surfaces, where they act as cytokine receptors and in the extracellular space where they form the structure of the extracellular matrix and serve as a storage reservoir for growth factors. Glycosaminoglycan degrading enzymes from *F. heparinum*: heparinase 1 (EC 4.2.2.7), heparinase 2, heparinase 3 (EC 4.2.2.8), chondroitinase AC (EC 4.2.2.5) and chondroitinase B modulate the interactions involved in cell proliferation and migration by i) releasing heparin binding growth factors and molecules from the extracellular matrix, thereby increasing their availability to adjacent cells for the stimulation of proliferation and migration, ii) degrading components of the extracellular matrix, thereby facilitating the mobility of cytokines, chemoattractants and cells, iii) removing chondroitin sulfate from cell surfaces, thereby increasing access to cell surface receptors and iv) inhibiting the proliferative response of cells to growth factors by removing the heparan sulfate component of their growth factor receptor complex.

Heparin binding growth factor-receptor interactions require the presence of a third component: heparan sulfate, which is present on cell surfaces, or can be added to the cells, or released lytically as a heparan sulfate fragment from the extracellular matrix. The addition of heparin or heparan sulfate degrading enzymes in the range of between 0.001 and 5 IU/ml promotes cell proliferation by co-releasing heparin binding growth factors and heparan sulfate fragments from the extracellular matrix and increasing their availability to adjacent cells.

Selectively removing heparan sulfate from cell surfaces while leaving the extracellular matrix intact, conversely, inhibits cell proliferation by down regulating the cell's response to growth factors. This is achieved by targeting heparin or heparan sulfate degrading activities to the cell surface. Targeting the heparin degrading activity can be achieved by genetically engineering a ligand binding functionality into the heparinase proteins, or by physically controlling the localized enzyme concentration through the method of administration. For example, permeable double balloon catheters can direct heparinases, preferentially, to exposed vascular smooth muscle cells in injured vessels. Preparation of Glycosaminoglycan Degrading Enzymes

Glycosaminoglycan lysase enzymes can be prepared by isolation from bacterial or mammalian cells, either those which naturally produce the enzymes or have been genetically engineered to produce the enzymes.

### Isolation of naturally Produced enzymes.

Glycosaminoglycan lyase enzymes can be purified from cultures of *Flavobacterium heparinum,* as follows. *F. heparinum* is cultured in 15 L computer controlled fermenters, in a variation of the defined nutrient medium described by Galliher et al., *Appl Environ. Microbiol.* 41 (2) :360-365, 1981. For fermentations designed to produce heparin lyases, semi-purified heparin (Celsus Laboratories) is included in the media at a concentration of 1.0 g/L as the inducer of heparinase synthesis. For fermentations designed to produce chondroitin lyases, chondroitin sulfate A (Sigma) is included in the media at a concentration of 1.0 g/L as the inducer of chondroitinase AC and chondroitinase B synthesis. For both types of fermentation, the cells are harvested by centrifugation and the desired enzymes released from the periplasmic space by a variation of the osmotic shock procedure described by U.S. Patent No. 5,169,772 to Zimmermann, et al. (1992).

Proteins from the crude osmolate are adsorbed onto cation exchange resin (CBX, J.T. Baker) at a conductivity of between one and seven µmho. Unbound proteins from the extract are discarded and the resin packed into a chromatography column (5.0 cm i.d. x 100 cm). The bound proteins elute at a linear flow rate of 3.75 cm·min⁻¹ with step gradients of 0.01 M phosphate, 0.01 M phosphate/0.1 M sodium chloride, 0.01 M phosphate/0.25 M sodium chloride and 0.01 M phosphate/ 1.0 M. sodium chloride, all at pH, 7.0 ± 0.1. Heparinase 2 elutes in the 0.1 M NaCl fraction while heparinases 1 and 3 elute in the 0.25 M fraction. Alternately, the 0.1 M sodium chloride step is eliminated and the three heparinases co-eluted with 0.25 M sodium chloride. The heparinase fractions are loaded directly onto a column containing cellufine sulfate (5.0 cm i.d. x 30 cm, Amicon) and eluted at a linear flow rate of 2.50 cm·min⁻¹ with step gradients of 0.01 M phosphate, 0.01 M phosphate/0.2 M sodium chloride, 0.01 M phosphate/0.4 M sodium chloride and 0.01 M phosphate/ 1.0 M. sodium chloride, all at pH, 7.0 ± 0.1. Heparinase 2 and 3 elute in the 0.2 M sodium chloride fraction while heparinase 1 elutes in the 0.4 M fraction. The 0.2 M sodium chloride fraction from the cellufine sulfate column is diluted with 0.01 M sodium phosphate to give a conductance less than 5 µmhos. The solution is further purified by loading the material onto a hydroxylapatite column (2.6 cm i.d. x 20 cm) and eluting the bound protein at a linear flow rate of 1.0 cm·min-1 with step gradients of 0.01 M phosphate, 0.01 M phosphate/0.35 M sodium chloride, 0.01 M phosphate/0.45 M sodium chloride, 0.01 M phosphate/0.65 M sodium chloride and 0.01 M phosphate/ 1.0 M. sodium chloride, all at pH, 7.0 ± 0.1. Heparinase 3 elutes in a single protein peak in the 0.45 M sodium chloride fraction while heparinase 3 elutes in a single protein peak in the 0.65 M sodium chloride fraction. Heparinase 1 is further purified by loading material from the cellufine sulfate column, diluted to a conductivity less than 5 µmhos, onto a hydroxylapatite column (2.6 cm i.d. x 20 cm) and eluting the bound protein at a linear flow rate of 1.0 cm·min-1 with a linear gradient of phosphate (0.01 to 0.25 M) and sodium chloride (0.0 to 0.5 M). Heparinase 1 elutes in a single protein peak approximately mid-way through the gradient.

The heparinase enzymes obtained by this method are greater than 98.5 % pure as estimated by reverse phase HPLC analysis (BioCad, POROS II). Purification results for the heparinase enzymes are shown in Table 1.

**TABLE 1: Purification of heparinase enzymes from Flavobacterium heparinum fermentations**

| **sample** | **activity (IU)** | **specific activity (IU/mg)** | **yield (%)** |
|---|---|---|---|
| fermentation | | | |
| heparin degrading | 94,500 | | 100 |
| heparan sulfate | | | |
| degrading | 75,400 | ND | 100 |
| osmolate heparin | | | |
| degrading | 52,100 | | 55 |
| heparan sulfate | | | |
| degrading | 42,000 | ND | 56 |
| cation exchange | | | |
| heparin degrading | 22,600 | | 24 |
| heparan sulfate | | | |
| degrading | 27,540 | ND | 37 |
| cellufine sulfate | | | |
| heparin degrading | 19,200 | | 20 |
| heparan sulfate | | | |
| degrading | 9,328 | 30.8 | 12 |
| hydroxylapatite | | | |
| heparinase 1 | 16,300 | 115.3 | 17 |
| heparinase 2 | 2,049 | 28.41 | 3 |
| heparinase 3 | 5,150 | 44.46 | 7 |

Osmolates obtained from *F. heparinum* fermentations induced with chondroitin sulfate A are subjected to centrifugation to remove cells and cell debris and the supernatant applied to a cation exchange column (5.0 cm x 30 cm, Sepharose^{™} S Big Beads, Pharmacia) at a linear flow rate of 10 cm·min⁻¹. The bound proteins are eluted at a linear flow rate of 5.1 cm·min⁻¹ with step gradients of 0.01 M phosphate, 0.01 M phosphate/0.25 M sodium chloride and 0.01 M phosphate/ 1.0 M. sodium chloride, all at pH, 7.0 ± 0.1. Chondroitinase activity elutes in the 0.25 M sodium chloride fraction which is further purified by diluting the chondroitinase containing fraction two-fold with 0.01 M sodium phosphate and applying the material onto a column containing cellufine sulfate (2.6 cm i.d. x 100 cm, Amicon) and eluting at a linear flow rate of 1.88 cm·min⁻¹ with a linear gradient of sodium chloride, 0.0 to 0.4 M. Chondroitinase AC primarily elutes at 0.23 to 0.26 M sodium chloride while chondroitinase B eluted at 0.27 to 0.3 M sodium chloride. Each fraction was diluted two-fold with 0.01 M sodium phosphate and applied to a hydroxylapatite column (2.6 cm i.d. x 30 cm). The bound proteins are eluted with a step gradient of 0.25 M sodium chloride followed by a linear gradient of 0.25 to 1.0 M sodium chloride all in 0.025 M sodium phosphate at pH 7.0 ± 0.1. Chondroitinase B elutes in the 0.25 M sodium chloride step while chondroitinase AC elutes at 0.85 to 0.95 M sodium chloride. The chondroitinase B fraction is diluted two-fold in 0.01 M sodium phosphate and applied to a strong cation exchange column (CBX-S, J. T. Baker, 1.6 cm i.d. x 10 cm). The bound material is eluted at a flow rate of 1.0 cm·min⁻¹ with a linear gradient from 0.125 to 0.325 M sodium chloride in 0.025 M sodium phosphate at pH 7.0 ± 0.1. Chondroitinase B elutes in a protein peak at 0.175 to 0.225 M sodium chloride and contains a minor contaminating protein of molecular weight 20,000 D. This protein is removed by gel filtration chromatography by loading the chondroitinase B sample onto a Superdex^{™} 200 column (1.0 x 30 cm, Pharmacia) and eluting with 0.05 M sodium phosphate, pH 7.2 at a linear flow rate of 1.25 cm·min⁻¹ and collecting the protein containing fractions. The chondroitinase AC fraction collected from hydroxylapatite chromatography is diluted three-fold in 0.01 M sodium phosphate and applied to a strong cation exchange column (CBX-S, J. T. Baker, 1.6 cm i.d. x 10 cm). The bound material is eluted at a flow rate of 1.0 cm·min⁻¹ with a linear gradient from 0.125 to 0.325 M sodium chloride in 0.025 M sodium phosphate at pH 7.0 ± 0.1. Chondroitinase AC elutes in a single protein peak at 0.175 - 0.225 M sodium chloride. Purification results for the chondroitinase enzymes are shown in Table 2.

**TABLE 2: Purification of chondroitinase enzymes from Flavobacterium heparinum fermentations**

| **sample** | **activity (IU)** | **specific activity (IU/mg)** | **yield (%)** |
|---|---|---|---|
| fermentation | | | |
| chondroitinase AC | 65,348 | 0.764 | 100 |
| chondroitinase B | 21,531 | 0.252 | 100 |
| osmolate | | | |
| chondroitinase AC | 39,468 | 1.44 | 60 |
| chondroitinase B | 15,251 | 0.588 | 71 |
| cation exchange | | | |
| chondroitinase AC | 27,935 | 9.58 | 43 |
| chondroitinase B | 13,801 | 4.731 | 64 |
| cellufine sulfate | | | |
| chondroitinase AC | 18,160 | 22.6 | 28 |
| chondroitinase B | 6,274 | 21.2 | 29 |
| hydroxylapatite | | | |
| chondroitinase AC | 14,494 | 146.8 | 22 |
| chondroitinase B | 3,960 | 65.62 | 18 |
| strong cation exchange | | | |
| chondroitinase AC | 9,843 | 211.4 | 15 |
| chondroitinase B | 4,104 | 167.2 | 18 |
| gel filtration | | | |
| chondroitinase B | 2,814 | 278.7 | 13 |

### Isolation of recombinant enzymes.

Glycosaminoglycan degrading enzymes also can be isolated from recombinant expression systems such as the heparinase 1 expression system described by Sasisekharan, et al., Proc. Natl. Acad. Sci. USA 90:8660-8664, 1993; the heparinase 2 and 3 expression systems disclosed in U.S. Patent application serial No. 08/258,639 "Nucleic Acid Sequences and Expression Systems for Heparinase 2 and Heparinase 3 Derived From *Flavobacterium heparinum"* by Su, et al., filed June 10, 1994; or the chondroitinase AC and B expression systems disclosed in U.S. Patent application serial No. 08/272,247 "Chondroitin Lyase Enzymes" by Bennett, et al., filed July 8, 1994, the teachings of which are incorporated herein. In these expression systems, the *F. heparinum* genes are isolated and cloned into plasmids downstream from an inducible promoter. The plasmids are introduced into *E. coli* and the expression of the desired enzyme directed by a suitable induction method such as temperature shift and addition of IPTG to the medium.

The enzymes can be recovered in a purified form by a modification of the methods described herein. Cell disruption is achieved by homogenization, sonication or enzyatic treatment to break the cell wall and release cytoplasmic components. If enzyme synthesis results in aggregation, the aggregate can then be dissolved by a denaturing agent, 3 to 6 M guanidine HCl or 4 to 8 M urea and the protein refolded by removal of the denaturing agent through dialysis or dilution. The refolded enzyme can be further purified using the liquid chromatographic methods described above.

### Construction of fusion proteins

Fusion proteins incorporating glycosaminoglycan degrading enzymes ligated to proteins with specific binding properties can be created by recombinant molecular biology techniques. By choosing an appropriate binding protein, the glycosaminoglycan degrading activity can be targeted to specific sites *in vivo.* For example, epidermal growth factor binds cell receptors expressed preferentially on the surface of smooth muscle cells as described by Pickering, et al., *J Clin Invest,* 91:724-729, 1993. Fusion proteins containing this moiety ligated to a heparinase protein direct heparin or heparan sulfate degrading activity to the surface of smooth muscle cells, thereby diminishing their response to available cytokines. This type of fusion protein is of value in combating disease states that result from overgrowth of smooth muscle cells such as the vascular conditions of atherosclerosis and re-occlusion of vessels following percutaneous transluminal coronary angioplasty.

Heparinase fusion proteins created by genetic engineering retain the binding and catalytic properties of heparinase and of the protein to which it is fused. For example, the gene for heparinase 1 was isolated from *F. heparinum* as described by Sasisekharan, et al., *Proc. Natl. Acad. Sci.* 90:3660-3664, 1993, and an Eco R1 restriction site was inserted 5' to the codon encoding the glutamine-21 residue by polymerase chain reaction. A fragment containing the heparinase 1 gene was prepared by digestion with restriction endonucleases; Eco R1 and Bam H1, and ligated to the Eco R1/Bam H1 cleaved pMALc2 plasmid (New England Biolabs). The resulting plasmid contained a hybrid gene encoding a 62,000 - 85,000 protein incorporating the maltose binding protein (MalB) fused 5' to the heparinase 1 gene. This plasmid was inserted into *Esherichia coli* HB101 cells using the calcium chloride mediated method described by Cohen et al., *Proc. Natl. Acad. Sci.* 69:2110-211. These cells exhibited heparinase activity under the control of the lac promoter, allowing synthesis of the fusion protein by addition of 0.1 mM of the inducing agent IPTG to the growth medium.

The HB101(pMALc2-HEP1Q21) cells were grown to a cell density of 1.0 g/L dry cell weight in 500 ml, M9 medium containing 0.1 mM IPTG at 37°C and concentrated by centrifugation, 10,000 g x 10 minutes. The cell pellet was suspended in 10 ml 0.025 M Tris, pH 7.7, and the cells disrupted by sonication using a Heat Systems Model XL2020, 4.5 minutes, power level 3, 30 second on 30 second off cycles. Cell debris was removed by centrifugation, 10,000 g x 10 minutes, and the supernatant applied to an amylose affinity resin column (1.0 i.d. x 2 cm, New England Biolabs). The bound protein was eluted with a step gradient of 0.025 M Tris containing 0.01 M maltose at pH 7.5. The fusion protein eluted in a protein peak which displayed a heparinase specific activity of 23.77 IU/mg.

The heparinase-maltose binding fusion protein also can be purified by standard protein separation techniques based on heparinase properties. Cell sonicates were fractionated by ammonium sulfate precipitation. Non-specific proteins were removed with a precipitation step at 1.7 M ammonium sulfate and the supernatant precipitated by raising the ammonium sulfate concentration to 3.2 M. The precipitated material contained the fusion protein and was resuspended in 0.025 M sodium phosphate, pH 6.5. The material was applied to a weak cation exchange column (1.6 i.d. x 10 cm, CBX, J.T. Baker) and eluted with sequential step gradients of 0.0 M sodium chloride, 0.01 M sodium chloride, 0.25 M sodium chloride and 1.0 M sodium chloride, all in 0.025 M sodium phosphate. The fusion protein eluted in the 0.25 M sodium chloride elution fraction and displayed a heparinase specific activity of 29.95 IU/ml. These two purification procedures demonstrate that functional heparinase fusion proteins can be made by genetically linking a protein with desired binding properties to the N-terminal end of the heparinase protein and the resulting fusion protein retains the functionality of both heparinase and the protein to which it is fused. Examples of other targeting molecules which specifically bind to receptors such as ECM molecules include fibronectin, laminin, tenascin, thrombospondin, and collagens.

During the past two decades, the base knowledge of cell adhesion and migration in extracellular matrices (ECMs) at the molecular level has expanded rapidly. Early efforts in this area of research concentrated on the adhesion-promoting ECM protein fibronectin (FN). Sequence analyses and peptide mapping of the FN cell-binding domain yielded a minimal sequence which maintained cell-binding activity in the tetrapeptide Arg-Gly-Asp-Ser (RGDS). The biological interaction of the RGDS sequence with cell-surface fibronectin receptors was revealed by demonstrating that synthetic RGDS-containing peptides in solution could competitively inhibit fibroblast cell spreading on fibronectin-coated substrates. After the RGD cell adhesion recognition site in fibronectin was identified, the sequences of other cell adhesion proteins were examined for related signals. Other proteins known to carry functional RGD sequences include the platelet adhesion proteins fibrinogen and von Willebrand factor, osteopontin, and laminin. These findings imply that RGD is a ubiquitous cell adhesion signal. The use of fibronectin as the affinity ligand yielded a receptor that was a heterodimer with a 160 kD α-subunit and a 140 kD β-subunit. Similar affinity chromatography experiments have yielded distinct heterodimeric RGD-directed receptors specific for vitronectin and a platelet receptor with affinities for fibrinogen and fibronectin. These RGD receptors, known as integrins, have a heterodimeric structure characteristic of RGD-directed receptors, with α-subunits ranging between 140 and 160 kD and β-subunits ranging between 90 and 140 kD. Integrins are characteristically membrane-spanning heterodimeric protein complexes consisting of an α-subunit and a β-subunit. Integrin complexes containing β₁ and β₃ subunits generally are involved in cell adhesion to the extracellular matrix, while the β₂ integrins are involved in cell-cell adhesion.

Other binding proteins can be antibodies or antibody fragments that recognize specific cell markers, hormones or other molecules that are bound by cell surface receptors. An example of a hormone bound by certain cell types is estrogen, which is bound to a greater degree by certain types of cancer cells. Another example is melanin, which is also present in higher concentrations of certain cancer cells. Antibodies to many specific cell surface markers are known.

### Protection of Proteins In Vivo

Methods for extending the *in vivo* half-life are known and routinely used, especially in the case of enzymes. Examples of suitable methods using attachment of polyethylene glycol moieties to the protein, which inhibits uptake by the reticuloendothelial system. Preparation and characterization of "peglyated" proteins is described by Lu, et al., *Pept*. *Res*. 6(3), 140-146, 1993; Delgado, et al., *Critical Rev. Ther. Drug Carrier Syst.* 9(3-4), 249-304, 1992, the teachings of which are incorporated herein.

### Preparation of Pharmaceutical Compositions

The enzymes can be administered topically, locally or systemically. Topical or local administration is preferred for greater control. The enzymes, alone or in combination, are mixed with an appropriate pharmaceutical carrier, then administered in an effective amount to produce the desired effect on the treated cells using methods known to those skilled in the art, for example, for topical application, by direct application to a site, or for local application, by means of injection or catheter.

Targeting and effective concentration dosages can be achieved by preparation of targeted enzymes as described above, or by the use of targeting vehicles, such as a catheter or polymeric delivery system, to achieve controlled site specific delivery of enzyme.

### Preparation of Heparinase Gels:

Glycosaminoglycan degrading enzymes can be mixed with a variety of common gels, creams or ointments to facilitate their application for treatment of dermal wounds. These gels or ointments can be administered alone or in a transdermal patch or bandage to facilitate penetration of an effective amount of enzyme to the cells which are to be treated.

### Administration of enzymes via controlled release matrices or injection:

Enzymes can also be formulated in a carrier for administration by injection, for example, in saline or an aqueous buffer, using standard methodology, or encapsulated in a polymeric matrix.
Encapsulation of enzymes in controlled release formulations is well known; materials include but not limited to liposomes, lipospheres, biodegradable polymeric matrices, and vesicles. These encapsulants are typically microparticles having a diameter from 60 nm to 100 microns, but preferably less than ten microns, and more preferably one micron or less in diameter.

Proteosomes are prepared from outer membrane proteins of the *Meningococcal* bacteria and been reported to bind proteins containing hydrophobic anchors by Lowell, et al Science, 240:800 (1988). Proteosome proteins are highly hydrophobic, reflecting their role as transmembrane proteins and porins. When isolated, their hydrophobic protein-protein interactions cause them to form naturally multimolecular, membraneous 60 to 1000 nm vesicles or membrane vesicle fragments, depending on the strength of the detergent used in their isolation. The enzyme can also be encapsulated within a proteoliposome as described by Miller et al., J. Exp. Med. 176:1739-1744 (1992) and incorporated by reference herein, as described above with reference to proteosomes.
Alternatively, the enzyme can be encapsulated in lipid vesicles such as Novasome^{™} lipid vesicles (Micro Vescular Systems, Inc., Nashua, NH).
Another carrier is described in PCT US90/06590 by Nova Pharmaceuticals, the teachings of which are incorporated herein, which is referred to as a liposphere, having a solid core and an outer shell layer formed of phospholipid.

The carrier may also be a polymeric delayed release system. Biodegradable synthetic polymers are particularly useful to effect the controlled release of enzymes. Microencapsulation has been applied to the injection of microencapsulated pharmaceuticals to give a controlled release. A number of factors contribute to the selection of a particular polymer for microencapsulation. The reproducibility of polymer synthesis and the microencapsulation process, the cost of the microencapsulation materials and process, the toxicological profile, the requirements for variable release kinetics and the physicochemical compatibility of the polymer and the antigens are all factors that must be considered. Examples of useful polymers are polycarbonates, polyesters, polyurethanes, polyorthoesters and polyamides, particularly those that are biodegradable.

A frequent choice of a carrier for pharmaceuticals is poly (d,l-lactide-co-glycolide) (PLGA). This is a biodegradable polyester that has a long history of medical use in erodible sutures, bone plates and other temporary prostheses, where it has not exhibited any toxicity. A wide variety of pharmaceuticals including peptides and antigens have been formulated into PLGA microcapsules. The PLGA microencapsulation process uses a phase separation of a water-in-oil emulsion. The compound of interest is prepared as an aqueous solution and the PLGA is dissolved in a suitable organic solvents such as methylene chloride and ethyl acetate. These two immiscible solutions are co-emulsified by high-speed stirring. A nonsolvent for the polymer is then added, causing precipitation of the polymer around the aqueous droplets to form embryonic microcapsules. The microcapsules are collected, and stabilized with one of an assortment of agents (polyvinyl alcohol (PVA), gelatin, alginates, polyvinylpyrrolidone (PVP), methyl cellulose) and the solvent removed by either drying in vacuo or solvent extraction.
Other means for encapsulation include spray drying, co-precipitation, and solvent extraction.

Enzymes can also be applied as films or implants, for example, to coat a tissue where growth is to be inhibited. Examples of materials used for controlled release which are administered as gels or films incorporating the agent to be released include Pluronics^{™} (BASF), copolymers of polyethylene oxide and polypropylene glycol.

### Means for Administration

The enzymes can be administered topically, as described above, or by injection. Typically, injection is performed using either a syringe or catheter. The advantage of the catheter is that material can be applied to surfaces such as the inside of blood vessels during a procedure such as angioplasty, where the goal is to inhibit restenosis by inhibiting the abnormal proliferation of cells that frequently follows the surgical procedure. Enzymes can also be administered simultaneously with surgery, so that healing of the wound is enhanced. Enzymes could also be administered during surgery to accelerate healing of the surgical wound. This could be accomplished by formulating the enzyme in a biocompatible gel or salve that would be applied directly to the wound site at the conclusion of the corrective procedure.

Glycosaminoglycan degrading enzymes can be applied intra-dermally to elicit an accelerated formation of new vessels in ischemic regions. Mechanistically, this is achieved by the dislodgement of growth factors from their extracellular storage reservoir where they are sequestered by heparan sulfate proteoglycans and by enhancing the mobility of cytokines and chemoattractants through the diseased tissue area.

The present invention will be further understood by reference to the following nonlimiting examples.

### Example 1: Preparation of topical enzyme compositions.

A 0.5 ml solution of 0.01 M sodium phosphate 0.4 M sodium chloride and 200 IU heparinase 1, purified as described herein, was mixed with either 9.5 ml gel consisting of 1% carboxymethyl cellulose (Sigma), 40 % USP glycerol and Nanaopure^{™} water or 9.5 ml of a carbomer based gel (carbomer^{™} 950, Keystone Laboratories).

A portion of each mixture was analyzed for heparinase activity using the spectrophotometric method described by Yang, et al., *J. Biol Chem.* 260(3):1849-1857, 1985. A modification of the agarose plate assay system for monitoring heparin degradation described by Zimmermann, et al. *Appl Environ. Microbiol,* 56(11):3593-3594, 1990, was incorporated to monitor the desorption of heparinase from various carriers. A solution containing 0.5 % USP sodium heparin (Celsus Laboratories) and 1.0 % purified agarose (Bio-Rad) in 0.25 M sodium acetate and 0.0025 M cacium acetate at pH; 7.0 ± 0.5, was mixed at 95 - 100°C, cooled to 45 - 60°C, poured in 3 ml portions into 5 ml plastic disposable cuvettes and allowed to solidify by cooling to room temperature. Heparinase solutions (0.5 ml, 20 IU/ml) and heparinase containing gels (0.3 - 0.7 ml) were applied to the top of the heparin/agarose gels and incubated at 37°C for 1 h. The heparinase formulations were discarded, a cylindrical cross section of the gels removed with a glass Pasteur pipet and the cylinders placed in a 2 % protamine sulfate solution (Sigma). After 4 - 12 h, a heparin-protamine precipitation was observed as an opaque white substance. The extent of heparinase desorption was determined by the depth of the clear zone located at the top of the excised cylindrical gels.

This experiment was repeated in the carboxymethyl cellulose/glycerol formulation using either 20 IU/ml chondroitinasese AC or 20 IU/ml chondroitinase B as the active ingredient and chondroitin sulfate A or dermatan sufate B as the test reagent. The results are shown in Table 3.

**TABLE 3: Enzymatic activity and desorption of heparinase 1 from pharmaceutical gel formulations.**

| **pharmaceutical carrier** | **enzyme (IU/ml)** | **activity** | **desorption (mm)** | |
|---|---|---|---|---|
| | | | **1 hour** | **4 hour** |
| PBS | heparinase 1 | 100 | 4 | ND |
| 4% polyethy- | | | | |
| lene oxide | heparinase 1 | 6.9 | 3 | ND |
| carbomer gel | heparinase 1 | 5.5 | 1 | ND |
| glycerol/CM | | | | |
| cellulose | heparinase 1 | 12.2 | 3 | 7 |
| PBS | chondroitinase AC | 16.4 | 3 | ND |
| glycerol/CM | | | | |
| cellulose | chondroitinase AC | 12.5 | 1 | 3 |
| PBS | chondroitinase B | 4.7 | 3 | ND |
| glycerol/CM | | | | |
| cellulose | chondroitinase B | 6.5 | 1 | 4 |

### Example 2: Preparation of a heparinase or chondroitinase bandage.

The three bacterial heparinases and two chondroitinases, purified as described herein, were placed in solutions containing 0.01 M sodium phosphate, 0.2 M sodium chloride, pH 7.0 and 35 IU/ml enzyme. Semi-solid gels consisting of 4 % polyethylene oxide (7.5 cm x 5 cm x 0.3 cm) were contacted with 6 ml enzyme solution for 3 h, during which time more than 70 % of the enzyme solution absorbed into the gel matrix.

The enzyme containing gels were then tested for bioavailability (desorption) by the protamine precipitation of glycosaminoglycan - agarose gels as described herein. Enzyme containing patches were allowed to absorb to glycosaminoglycan-agarose gels for 90 minutes at 37°C before being transferred to a fresh agarose gel. The procedure was repeated for a total period of 7.5 hours. Semi-solid gels consisting of 4 % polyethylene oxide (7.5 x 5 x 0.3 cm) were soaked in 6 to 8 ml heparinase 1 at a concentration of between 35 and 60 IU/ml for three hours during which time the enzyme was absorbed into the matrix. The matrices were applied to 1 % agarose gels containing 0.05 % heparin and incubated at 37°C. Enzyme containing gels were transferred to fresh agarose gels each 90 minutes for a total of 7.5 hours. After incubation the agarose gels were contacted with 2.0 % protamine sulfate to precipitate unfractionated glycosaminoglycan. Penetration of the enzymes was observed by measuring the depth of the clear zone in the precipitated agarose gels. The results are illustrated in Figure 2.

### Example 3: Release of Growth Promoting Activity from Extracellular Matrix

*Flavobacterial* heparin degrading enzymes can dislodge substances exhibiting growth promoting activities from extracellular matrices. Primary endothelial cells were isolated from bovine corneal tissue and maintained in DMEM containing; 10 % fetal calf serum, and 5 % calf serum. Cells from confluent petri dishes were diluted 10-fold and grown in DMEM containing 10 % fetal calf serum, 4 % dextran and 5 % calf serum, in 96-well plates for 12 to 14 days and were supplemented with FGF-2 at the rate of 0.5 ng/ml-day. The endothelial cells were removed by treatment with a solution containing 0.5 % Triton and 0.02 M sodium hydroxide in phosphate buffered saline for 0.5 to 5 minutes, followed by three washes with phosphate buffered saline. This procedure yields plates coated with a layer of extracellular matrix which is stable for two years when stored at 4°C in phosphate buffered saline.

Varying amounts of the glycosaminoglycan degrading enzymes, purified as described herein, were added to the extracellular matrix in 0.2 ml/well containing 0.16 % fetal calf serum - DMEM. Contacting with the glycosaminoglycan degrading enzymes was allowed to take place for 1 hour at 37°C. The supernatants from these enzyme-extracellular matrix reaction mixtures were then tested for mitogenic activity by determining the incorporation of ³H-thymidine by quiescent balb/c 3T3 fibroblasts as described by Vlodavsky et al., *Proc. Natl. Acad. Sci.* 84:2292-2296, 1987.

Extracellular matrices formed in vitro from a primary endothelial cell line were treated with either heparinase 1, 2 or 3 at a concentration of 0.1 IU/ml, chondroitinase AC at a concentration of 1.0 IU/ml or chondroitinase B at a concentration of 0.5 IU/ml for 60 minutes. Reaction supernatants were tested for the presence of mitogenic activity by a thymidine incorporation assay. The results are shown in Figure 3.

### Example 4: Heparin and heparan sulfate degrading enzymes can also be used to release growth promoting activity from intact animal tissues.

Bovine corneas were harvested from cows at the time of slaughter. Each cornea was dissected into two equal sections and each section placed in 0.4 ml, DMEM. Heparinase at 0.1 IU/ml was added to one of the corneal sections and incubated at 37°C for 20 minutes. The remaining section from the same cornea served as the control. 20 µl aliquots from each reaction were transferred to 96-well plates containing starved 3T3 fibroblasts in a total volume of 200 µl in DMEM containing 0.2 % fetal calf serum. ³H-thymidine was added to each well and the cells incubated for 48 hours at 37°C.

Bovine corneas were harvested, dissected into two equal portions and treated with either heparinase 1, 2 or 3 at a concentration of 0.1 IU/ml. Reaction supernatants were tested for the presence of mitogenic activity by incorporation of ³H-thymidine determined by the method of Vlodavsky, et al. The results are shown in Figure 4.

### Example 5: Treatment of Extracellular Matrix by Glycosaminoglycan Lyases.

Glycosaminoglycan degrading enzymes alter the extracellular matrix by cleaving the glycosaminoglycan components of the extracellular matrix proteoglycan. Preparation of extracellular matrix with ³⁵S-sulfate containing proteoglycan and subsequent digestion of this radiolabelled matrix with *Flavobacterial* glycosaminoglycan degrading enzymes allows a quantitative assessment of the effect of the enzymes. ³⁵S-sulfate containing extracellular matrix was produced by seeding dishes with primary bovine corneal endothelial cells grown to confluence in DMEM with 10 % fetal calf serum and 5 % calf serum diluted 10-fold into Fisher medium supplemented with 10 % fetal calf serum, 5 % calf serum, 4 % dextran, and 25 µCi/ml Na₂³⁵SO₄ and cultured for 12 to 14 days with the addition of 0.5 ng/ml-day FGF-2. The endothelial cells were removed from the radiolabelled extracellular matrix by treatment with a solution containing 0.5 % Triton, 0.02 M sodium hydroxide in phosphate buffered saline for 0.5 to 5 minutes, followed by three washes with phosphate buffered saline.

Extracellular matrix containing ³⁵S sulfate in the glycosaminoglycan portion was treated with phosphate buffered saline or heparinases 1, 2 or 3, or chondroitinases AC or B at a concentration of 0.1 IU/ml in 1 ml/well dishes containing phosphate buffered saline, and the digestion was allowed to proceed for 0.5 hour at 37°C. The amount of glycosaminoglycan released was determined by measuring the radiolabelled sulfate released to the supernatant with a Packard 1600 TR liquid scintillation counter. An estimate of 80,000 cpm was the total amount of radiolabelled sulfate contained in each reaction. The results are shown in Figure 5.

The action of the *Flavobacterial* heparin degrading enzymes is extremely rapid, and the generation of ³⁵S-sulfate labeled material occurs seconds after their addition to radiolabelled extracellular matrix as described above. In contrast, an equal amount of mammalian heparanase isolated from human placenta shows a 15 to 20 minute lag time after addition to the radiolabelled matrix before any measurable increase in the level of soluble ³⁵S-sulfate labeled material is detected. This observation further differentiates the mammalian and bacterial enzymes.

While treatment of the extracellular matrix with glycosaminoglycan degrading enzymes alters the glycosaminoglycan component of the extracellular matrix proteoglycan, the overall structural integrity of the matrix remains unchanged as viewed by electron microscopy. Although structurally intact, enzymatically treated extracellular matrix exhibits enhanced permeability to macromolecules. This increased permeability can be demonstrated by examining the ability of the *Flavobacterial* glycosaminoglycan degrading enzymes to facilitate the passage of 25 nucleotide bases up to 2 Kb nucleotide fragments across a 0.45 micron pore polyethylene terephthalate (PET) membrane coated with extracellular matrix. Primary bovine corneal endothelial cells maintained as described above are diluted 1:10 from confluent dishes and seeded onto 0.45 micron pore PET membrane tissue culture inserts (Falcon) in DMEM supplemented with 10 % fetal calf serum, 5 % calf serum, 4 % dextran, and cultured for 12 to 14 days with the addition of 0.5 ng/ml-day FGF-2. The endothelial cells are removed as described above, and the extracellular matrix coated PET inserts treated with either heparinase 1, 2, or 3 at a concentration 0.1 IU/ml, or with either chondroitinase AC or B at a concentration of 1 IU/ml in phosphate buffered saline at 37°C for 1 hour and rinsed three times with phosphate buffered saline.

The enzymatically treated extracellular matrix coated PET inserts, along with an untreated extracellular matrix coated PET insert and an uncoated PET insert, are placed into 12 well dishes and 2 ml of phosphate buffered saline added to each well. Radiolabelled macromolecules are added inside each PET insert, and 100 µl aliquots of phosphate buffered saline solution in the well surrounding the PET insert taken after a 15 minute incubation at 37°C. Aliquots are assayed for ³²P-containing material by liquid scintillation in a Packard 1600 TR scintillation counter.

### Example 6: Treatment of Cell Surface with Glycosaminoglycan lyases.

Glycosaminoglycan degrading enzymes can attenuate a cell's response to growth factors by cleaving the glycosaminoglycan component of cell surface proteoglycans. Vascular smooth muscle cells were grown in 96 well plates in DMEM supplemented with 10 % fetal serum until confluent. The cells were treated with either heparinase 1, 2 or 3 or chondroitinase AC at a concentration of 0.1 IU/ml for 1 hour at 37°C, then chilled on ice and washed twice with an incubation medium comprised of 0.025 M HEPES, 0.002 M Tris and 0.1 % BSA in DMEM at pH 7.5. The cells were suspended in 0.25 ml incubation buffer containing 5 ng ¹²⁵I-FGF-2 (0.5 µCi) and incubated at 4°C for 2 hours. Adsorption of FGF-2 to cell surface glycosaminoglycan was determined by washing the cells with an elution buffer consisting of 0.025 M HEPES and 2 M sodium chloride at pH 7.4, and measuring the recovered ¹²⁵I with a gamma-counter (Wallac, Model 1740).

Balb/C 3T3 fibroblasts were treated with 0.1 IU/ml heparinases 1, 2 or 3, or chondroitinase AC and exposed to ¹²⁵I-FGF-2. The amount of FGF-2 adsorbed to the cell surface glycosaminoglycan was determined by extracting the glycosaminoglycan bound fraction in 0.025 M HEPES, 2.0 M sodium chloride and measuring FGF-2 using a gamma counter and is expressed as a percentage of FGF-2 bound to untreated cells. The results are shown in Figure 6.

### Example 7: Control of proliferation of endothelial cells using glycosaminoglycan treatment.

Glycosaminoglycan degrading enzyme treatment of cell surfaces can either enhance growth factor binding as in the case of chondroitin degrading enzymes, or inhibit growth factor binding as in the case of heparin and heparan sulfate degrading enzymes. The removal of cell surface heparan sulfate can be compensated by heparin or heparan sulfate fragments released from the extracellular matrix by enzymatic treatment.

Treated vascular smooth muscle cells were exposed to 0.1 IU/ml heparinase 2 at 37°C for 20 minutes. Treated matrix was exposed to 0.1 IU/ml heparinase 2 at 37°C for 20 minutes. After enzymatic treatment, the cells were washed with 0.1 ml PBS and exposed to 50 µl matrix supernatant.

³H-thymidine was included in the incubation and proliferation determined as described by Vlodavsky et al., *Proc. Nat. Acad. Sci. (USA)* 84:2292-6 (1987), *Trends Biochem. Sci.* 16:268-271 (1991). Proliferation of vascular smooth muscle cells was monitored by thymidine incorporation and is expressed as a ratio of cells exposed to enzyme released material to that of untreated matrices for a) untreated ECM, untreated cells, b) heparinase 2 treated ECM, untreated cells, and c) heparinase 2 treated ECM, treated cells. The results are shown in Figure 7.

The results show that if one separates cell matrix from cell surface, one will knock out receptor by treating the surface and release growth promoting activity by treating matrix, and that if one treats the matrix and the cell surface, growth promotion is observed since the matrix releases growth factor that compensates for the loss of heparin binding receptor.

### Example 8: Evaluation of local administration of heparinase to enhance revascularization.

A rabbit hind limb ischemic model described by Pu, et al., *Circulation* 88:208-215, 1993, was used to evaluate the effectiveness of heparinase 1 on restoring vascularization. Three treatment groups were studied (N = 4). Rabbits in each group recieve either saline control, FGF-2 at 100 mg-day⁻¹, or heparinase 1 at 100 IU-day⁻¹. Ischemia was surgically induced in the left hind limb and the compounds were administered for 10 days beginning on the 11th day following surgery. Rates of vascularization were monitored by measuring the blood pressure in both limbs with a Doppler flowmeter and calculating the ratio of blood flow in the ischemic limb to that of the control (untreated limb).

Heparinase 1 and FGF-2 accelerated both the increase of blood pressure ratio as well as the extent of blood pressure ratio achieved 30 days post-treatment. At post-operative day 40, angiograms were performed to determine new vessel formation. The results are shown in Table 4.

**TABLE 4: Treatment of ischemic hind limb**

| | **blood pressure ratio (IU/ml)** | | | **vessel formation** |
|---|---|---|---|---|
| **agent** | **day 10** | **day 20** | **day 30** | |
| PBS | 0.28 | 0.40 | 0.49 | 8.00 ± 1.00 |
| FGF-2 | 0.19 | 0.55 | 0.62 | 15.50 ± 2.38 |
| heparinase 1 | 0.30 | 0.60 | 0.71 | 22.50 ± 3.56 |

The data indicate the potential utility of compositions containing one or a combination of the *Flavobacterium* heparinum derived glycosaminoglycan degrading enzymes for accelerating tissue repair in humans.

### Example 9: Release of Growth promoting activity from Extracellular Matrix.

Extracellular matrices (ECM), prepared as described in Example 3, were treated with either heparinase 1, 2, or 3 at a concentration of 0.1 IU/ml or chondroitinase AC at a concentration of 1.0 IU/ml, for 10 minutes at 37°C. Untreated control samples were treated with a control solution which did not contain enzyme. A volume of 0.01 ml of each reaction supernatant was assayed for the presence of mitogenic activity by transfer to quiescent balb/C 3T3 fibroblasts using the proliferation assay described in Example 7. The results are presented in Figure 8.

Each of the test treatments, heparinase 1, 2, 3, or chondroitinase AC, released soluble material from the ECM which stimulated fibroblast proliferation above the level obtain with untreated ECM.

### Example 10: Treatment of the Cell Surface and Extracellular Matrix with heparinase.

Heparinase administered to a wound *in vivo* will act not only on the extracellular matrix, but also on the glycosaminoglycans on the cell surface of cells participating in the wound healing process. In order to model this effect *in vitro,* quiescent balb/C 3T3 fibroblasts were treated with heparinase 3 in the same manner as the extracellular matrix prior to receiving the extracellular matrix reaction supernatant. Both extracellular matrices and quiescent balb/C 3T3 fibroblasts were treated with heparinase 3 at a concentration of 0.1 IU/ml for 10 min at 37°C, after which time the enzyme-containing supernatant was removed from the cells and replaced with DMEM containing 0.2% fetal calf serum. Following this treatment, proliferation of 3T3 fibroblasts was determined by monitoring ³H-Thymidine incorporation. The results are presented in Figure 9.

Higher proliferation responses were seen when the fibroblasts were treated with enzyme prior to receiving the treated ECM. The results support the use of heparinase 3 at the wound site to stimulate wound healing.

### Example 11: Heparinase-mediated release of growth promoting activity from intact animal tissues.

Bovine corneas were harvested from cows at the time of slaughter and treated with various concentrations of heparinase 3. Using 3 bovine corneas for each concentration, the corneas were placed in sterile 24-well tissue culture dishes such that only the interior (Descemet's) membrane was exposed, and 0.2 ml of phosphate buffered saline was added to each cornea. Heparinase 3 was then added to a final concentration of 0.01, 0.1 or 1.0 IU/ml, and the digestion allowed to proceed for 5 minutes at 37° C. One control set of 3 corneas received no enzyme. Aliquots of 0.045 ml reaction supernatant was then transferred from each cornea to quiescent balb C 3T3 fibroblasts. Proliferation of the fibroblasts was determined using the proliferation assay described in Example 7. The results are presented in Figure 10.

All three concentrations of heparinase 3 cause the release of proliferation stimulating compounds from the Bovine cornea, with the greatest effect resulting from treatment with 0.1 IU heparinase 3/ml.

### Example 12: Heparinase-mediated release of bFGF from Cells and Extracellular Matrix in vitro.

To determine the relative effectiveness of heparinase 1, 2, and 3 to release heparin-binding growth factors from tissues and extracellular matrices, supernatants from heparinase 1, 2, or 3 digested cells or matrix were assayed for the presence of bFGF. Confluent bovine endothelial and smooth muscle cells maintained in 10 cm tissue culture dishes were incubated for 1 hour at 37°C in 2 ml of phosphate buffered saline containing 0.1 IU/ml heparinase 1, 2, or 3. Extracellular matrix, prepared as described in Example 3, was treated identically. Aliquots were removed for determination of bFGF concentration using the Quantikine^{™} ELISA (R&D systems) for human bFGF. The results are presented in Figure 11.

Heparinase 3 caused the greatest amount of bFGF to be released from all three cell types, followed by heparinase 2, then heparinase 1. ECM release more bFGF than bovine endothelial cells and bovine smooth muscle cells with all three enzymes tested.

### Example 13: Heparinase-mediated release of bFGF from Cells and Extracellular Matrix in vitro.

Bovine aorta smooth muscle cells (passages 1-8) were grown to near confluency in DMEM (high glucose - DMHG) supplemented with 10% fetal serum and 100 units/ml of penicillin/streptomycin, in 96 well dishes, at 37°C, in a 7.5 % CO₂ environment. The cells were starved for 3.5 to 4 days by exchanging the growth medium for DMHG and 2% BSA. After the starvation period, the cells were treated with a solution of DMHG and 0.5% BSA containing heparinase, 1, 2 or 3, at 0.1 or 0.5 IU/ml, at 37°C, for 10 min. The enzyme solution was removed and the wells were washed three times with phosphate buffered saline with calcium and magnesium, (PBS + Ca + Mg). 180 µl of DMHG, 0.5% BSA and 1.1 µCi/ml ³H-thymidine was added to each well. In addition, 20 µl of 20 ng/ml bFGF in DMHG and 0.5% BSA was added to the induced wells, and 20 µl of DMHG and 0.5% BSA was added to control wells. The cells were incubated for 48 hr, as described above. After 48 hr, the medium was removed and the wells were washed once with PBS + Ca + Mg, once with 200 µl of 100% methanol, two times with 5% TCA, and two times with water. After these washing steps, 100 ul of 0.2 N NaOH was added to each well. After 5 min at room temperature, the contents of the wells were added to vials containing scintillant and the amount of incorporated ³H was measured. The results from two separate experiments were averaged and are shown in Figure 12.

### Example 14: Basic FGF receptor activation by heparin sulfate degradation fragments released from cells and extracellular matrix.

### Materials and Methods.

Recombinant human bFGF was provided by Takeda Chemical Industries (Osaka, Japan). Sepharose^{™} 6B was from Pharmacia (Uppsala, Sweden). Sodium heparin from porcine intestinal mucosa (PM-heparin, Mr 14,000, anti-FXa 165 IU/mg) was obtained from Hepar Industries (Franklin, Ohio). Bacterial (*Flavobacterium heparinum*) heparinase I (EC 4.2.2.7), 2 and 3 were produced by IBEX Technologies, (Montreal, Canada). Heparan sulfate degrading endoglycosidase (heparanase) was purified from human placenta. Enzyme purification involved ammonium sulfate precipitation and sequential chromatographies over carboxymethyl Sepharose, heparin Sepharose and Con A Sepharose.

*Cells.* Smooth muscle cells (SMC) were isolated from the bovine aortic media as described by Castellot, J.J., et al., *J. Cell Biol.* 102, 1979-1984 (1986); Schmidt, A., et al., *J. Biol. Chem.* 267, 19242-19247 (1992). Briefly, the abdominal segment of the aorta was removed and the fascia cleaned away under a dissecting microscope. The aorta was cut longitudinally, and small pieces of the media were carefully stripped from the vessel wall. Two or three such strips with average dimensions of 2 mm³ were placed in 100 mm tissue culture dishes containing DMEM (4.5 g glucose/liter) supplemented with 10% FCS, 100 U/ml penicillin and 100 µg/ml streptomycin. Within 7-14 days, large patches of multilayered cells migrated from the explants. Approximately 1 week later, the cells were subcultured into 100 mm tissue culture plates (4-6 x 10⁵ cells/plate). The cultures (passage 38) exhibited typical morphological characteristics of vascular SMC and the cells were specifically stained with monoclonal antibodies that selectively recognize muscle form of actin (HS-35). This antibody does not recognize endothelial cells or fibroblasts.

Cultures of bovine corneal endothelial cells were established from steer eyes as described by Gospodarowicz, D., et al., *Exp. Eye Res.* 25, 75-89 (1977). Stock cultures were maintained in DMEM (1 g glucose/liter) supplemented with 10% newborn calf serum, 5% FCS, 50 U/ml penicillin, and 50 µg/ml streptomycin at 37°C in 10% C0₂ humidified incubators. Bovine aortic endothelial cells were cloned and cultured as.:described by Gospodarowicz, D., et al., *Proc. Natl. Acad. Sci . USA,* 73, 4120-4124 (1976). Recombinant bFGF (1 ng/ml) was added every other day until the cells were nearly confluent. Clone F32 of BaF₃ cells (Ornitz, D.M., et al., *Mol. Cell Biol.* 12, 240-247 (1992)) was provided by Dr. D. Ornitz (Department of Molecular Biology, Washington University in St. Louis). Cells were grown in RPM1 1640 medium supplemented with 10% new born calf serum, 10% interleukin-3 conditioned medium produced by X63-lL3 cells, L-glutamine and antibiotics. F32 cells were obtained following transfection of BAF₃ cells with Mo/mFR1/SV expression vector and selection in medium containing bFGF plus heparin, yielding colonies which express the mouse FGF receptor 1 mRNA (mFR1) as described by Ornitz, D.M., et al., *Mol. Cell Biol.* 12, 240-247 (1992).

*Cell proliferation.* F32 cells were washed twice with RPM1 1640 medium. Cells (2 x 10⁴/well/0.2 ml) were plated in 96 well microtiter plates in the absence and presence of 5 ng/ml bFGF and increasing concentrations of HS degradation fragments released from cells and ECM by heparinase 1, 2 or 3. 48 h later, 1 µCi of ³H-thymidine was added per well, the cells were incubated for another 6 h and then collected with a PHD Cell Harvester^{™}. Incorporated thymidine was determined by liquid scintillation counting.

*Preparation of dishes coated with ECM.* Bovine corneal endothelial cells were dissociated from stock cultures (second to fifth passage) with STV and plated into 4- ell plates at an initial density of 2 x 10⁵ cells/ml. Cells were maintained as described above except that 5% dextran T-40 was included in the growth medium and the cells were maintained without addition of bFGF for 12 days. The subendothelial ECM was exposed by dissolving for 5 min at room temperature the cell layer with PBS containing 0.5% Triton X-100 and 20 mM NH₄OH, followed by four washes in PBS. The ECM remained intact, free of cellular debris and firmly attached to the entire area tissue culture dish. For preparation of sulfate-labeled ECM, corneal endothelial cells were plated into 4-well plates and cultured as described above. Na₂[³⁵S]0₄ (540-590 mCi/mmol) was added (40 µCi/ml) one day and 5 days after seeding and the cultures were incubated with the label without medium change. Ten to twelve days after seeding, the cell monolayer was dissolved and the ECM exposed, as described above. Degradation of sulfate labeled ECM by bacterial heparinases was determined as described. Ishai-Michaeli, R., et al., *Cell Reg.* 1, 833-842 (1990); Bar-Ner, M., et al., *Blood* 70, 551-557 (1987); Vlodavsky, I., et al., *Cancer Res.* 43, 2704-2711 (1983). Briefly, ECM was incubated for 24 h, at 37°C, pH 6.2, with heparinase 1, 2 or 3 and sulfate labeled material released into the incubation medium was analyzed by gel filtration on a Sepharose 6B column. Intact heparan sulfate proteoglycans (HSPG) were eluted next to the void volume (Kav<0.2) and HS degradation fragments eluted with 0.5<Kav<0.8.

### Results.

*Degradation of sulfate labeled ECM and release of ECM-bound mitogenic activity by heparinase 1,2 and 3.* Degradation of HS in ECM was studied by incubating for 1 h at 37°C heparinase 1, 2 or 3 (0.1 U/ml) with metabolically sulfate labeled ECM produced by cultured bovine corneal - endothelial cells. Sulfate labeled degradation products released into the incubation medium were analyzed by gel filtration on Sepharose^{™} 6B. While intact HSPG is eluted next to the void volume of the column, labeled degradation fragments of HS side chains were eluted more toward the Vₜ of the column (0.5<Kav<0.8). As demonstrated in Figure 13A, incubation of ECM with each of the bacterial enzymes resulted in release of low Mr sulfate labeled degradation products (peak 11, fractions 20-30). The HS nature of these fragments was verified by their susceptibility to deamination with nitrous acid and resistance to further degradation deamination with papain or chondroitinase ABC. The three enzymes yielded different elution patterns reflecting different sizes of degradation fragments. Heparinase 1 yielded a broad distribution of fragments (0.4<Kav<0.6) with an average MW higher than that of fragments released by heparinase 3 (Kav-0.65) and heparinase 2 (Kav-0.8) (Figure 13). Heparinase 2 degrades the ECM HS into small fragments containing as little as 2-6 sugar units migrating close to the Vₜ of the column.

Material released from ECM by heparinase 1, 2 and 3 was added to growth arrested 3T3 fibroblasts and tested for its ability to stimulate DNA synthesis in these cells. As demonstrated in Figure 13B, material released from ECM by heparinase 2 and, to a somewhat lower extent, by heparinase 3, was highly mitogenic to 3T3 fibroblasts as compared to that released under the same conditions by heparinase 1. In fact, mitogenic activity released by heparinase 1 was only slightly higher than that released from ECM during incubation with PBS alone. The spontaneous release of both HSPG and mitogenic activity from ECM incubated with PBS alone is attributed to proteolytic enzymes such as tissue plasminogen activator (tPA), urokinase, and gelatinase A, residing in the ECM. Heparinase 1, 2 and 3 were devoid of any mitogenic activity as indicated by the lack of growth promoting activity when the enzymes were incubated on regular tissue culture dishes rather than ECM.

The difference in mitogenic activity released from ECM by heparinase 1, 2 and 3 was not due to a difference in their capacity to degrade the ECM substrate since greater than 90% of the ECM sulfate labeled material was released by each of the enzymes with less than 10% of the radioactivity remaining associated with the ECM, as shown by Figure 13C. Similarly, each of the three enzymes released greater than 90% of ¹²⁵I-bFGF that was first bound to the ECM. Moreover, a simultaneous incubation of sulfate labeled ECM with heparinase 1 and 3, or sequential additions of a second dose of the same or another enzyme yielded only a slight increase (less than 15%) in the amount of released HS degradation fragments and mitogenic activity.

### Growth promoting activity of HS fragments released from cells and ECM by heparinase 1, 2 and 3

A cytokine dependent lymphoid cell line engineered to express the mouse FGF receptor 1 (Ornitz, D.M., et al., *Mol. Cell Biol.* 12, 240-247 (1992)) was applied to the cells to investigate whether HS degradation fragments released from cells and ECM by heparinase 1, 2 & 3 can replace the need for heparin or heparan sulfate in enabling bFGF-induced mitogenesis in this cell system. It has been previously demonstrated that BaF₃ cells transfected to express mFR1 demonstrate a dose dependent response to bFGF with an absolute requirement for heparin. The F32 cells in these experiments were incubated with excess recombinant bFGF (5 ng/ml) so that any growth promoting effect induced by ECM degradation products could be attributed to fragments of heparan sulfate rather than to the relatively negligible amount (less than 0.5 ng/ml) of ECM-bound bFGF released under the same conditions. Vascular endothelial and smooth muscle cells (EC and SMC, respectively) as well as intact subendothelial ECM were incubated (1 h, 37°C) with 0.1 U/ml of heparinase 1, 2 or 3. Increasing amounts of the incubation media were then added to F32 cells in the presence of 5 ng/ml bFGF. Forty-eight hours later, ³H-thymidine was added for 6 h, followed by cell harvesting and measurement of ³H-thymidine incorporation.

Pretreatment of vascular endothelial and smooth muscle cells with heparinase 3 resulted in release of HS degradation fragments. In contrast, fragments released by heparinase 1 or 2 had no or very small effect, respectively, as shown by Figure 14. Similar studies performed with ECM revealed little or no stimulation of bFGF mediated cell proliferation by fragments released by heparinase 1, 2 or 3, above the basal ³H-thymidine incorporation obtained in the presence of bFGF and each of the bacterial enzymes alone.

In performing these control experiments, a slight stimulation of F32 cell proliferation induced by heparinase 3 alone, or following preincubation of heparinase 3, but not heparinase 1 or 2, in regular tissue culture dishes in the absence of cells or ECM was observed. As demonstrated in Figure 15, this stimulation was 3-4 fold lower than that induced by medium taken from heparinase 3 treated vascular SMC. Unlike the .. effect of heparin shown in Figure 14A and cell surface derived HS degradation fragments, the effect of heparinase 3 was abolished by heat inactivation for 10 min at 95°C prior to its addition to the F32 lymphoid cells (Figure 14B) and regardless of whether the heparinase 3 enzyme was first incubated on top of regular tissue culture plastic or ECM. This result indicates that the enzyme must be active and/or retain its native configuration to induce a mitogenic response. In an attempt to elucidate whether the heparinase 3 enzyme is releasing stimulatory HS-like fragments from the F32 cell surface, F32 cells were first treated with heparinase 3 (30 min, 0.1 U/ml, 37°C) and the supernatant with or without heat inactivation tested for its stimulatory effect on fresh, untreated F32 lymphoid cells. Again, ³H-thymidine incorporated was stimulated by heparinase 3, regardless of whether the enzyme was first incubated with F32 cells, and this stimulation was abolished by heat inactivation. In other experiments, the heparinase 3 enzyme was applied onto DEAE cellulose to remove traces of heparin which may have contaminated the enzyme. As demonstrated in Figure 14B, this treatment had no effect on the stimulatory activity of heparinase 3, but completely abolished the effect of standard heparin (Figure 14A). Altogether, these control experiments suggest that the native heparinase 3 enzyme itself is capable of stimulating bFGF receptor binding and activation in the F32 cell system.

### Release of ECM- and cell surface-bound bFGF

As demonstrated by Figure 13B, exposure of ECM to heparinase 2 and 3 and to a much lesser extent to heparinase 1, resulted in release of growth promoting activity towards growth arrested 3T3 fibroblasts. The actual amounts of bFGF released from cells and ECM heparinase 1, 2 and 3 were determined by an immunoassay (R & D Quantikine^{™} human bFGF). As shown in Table 5, the amount of bFGF released from ECM by heparinase 3 was about 2.5 and 15 fold higher than that released by heparinase 2 and heparinase 1, respectively, in correlation with the mitogenic activity exerted by the respective incubation media (Figure 13B). The amount of ECM-bound bFGF susceptible to release by heparinase 3 was 6-7 fold higher than that available on the surface of vascular endothelial and smooth muscle cells (Table 5). The amounts of bFGF released from vascular EC by heparinase 1 and 2 were higher than those released from vascular SMC.

The ability of three bacterial heparin/HS degrading enzymes to release from cells and ECM both HS-bound bFGF and HS degradation fragments that promote the mitogenic activity of bFGF has been compared. Actual measurements of released bFGF and stimulation of ³H-thymidine incorporation in growth arrested 3T3 fibroblasts and in HS deficient F32 lymphoid cells, clearly indicated that heparinase 3 was the most active growth promoting enzyme. The superiority of heparinase 3 was best demonstrated in the F32 cell system. In this system HS degradation fragments are evaluated for their ability to bind and present bFGF to its high affinity tyrosine kinase receptor, resulting in receptor activation and cell proliferation. It was found that HS fragments released from cell surfaces by heparinase 3, but not heparinase 1 or 2, were capable of serving as accessory receptors participating in a dual receptor mechanism characteristic of bFGF and other members of the heparin binding family of growth promoting factors. In contrast, little or no such activity was associated with HS fragments released by heparinase 3 from the subendothelial ECM. This result substantiates previous observations indicating that while HS in ECM provides a rather inert storage depot for bFGF in the vicinity of responsive cells, HS on cell surfaces may play a more active role in the actual displacement of ECM-bound bFGF and its subsequent presentation to high affinity cell surface receptor sites (Bernfield, M., et al., Ann. *N.Y. Acad. Sci.* 638, 182-194 (1991)).

These studies demonstrate the advantage of applying heparinase 3 as compared to heparinase 1 and 2 in releasing i) the highest amount of ECM-bound bFGF, and ii) HS degradation fragments capable of promoting bFGF receptor binding and activation in HS deficient cells. Surprisingly, control experiments applying each of the bacterial enzymes alone revealed that heparinase 3 itself stimulated the growth promoting activity of bFGF in the F32 cell system. Unlike the effect of heparin and HS degradation fragments, this stimulation was abolished following heat inactivation and was not removed by DEAE cellulose, indicating induction by the heparinase 3 protein rather then by heparin-like molecules that may be associated with the heparinase 3 preparation.

### Example 15: Wound healing in normal and impaired rat models.

The effectiveness of heparinase 3 to stimulate wound healing *in vivo* was tested using a rat impaired immune model, Mustoe, et al., *Science* 237: 1333-1326 (1987). Sprague-Dawley rats were wounded by making a 5.0 cm linear incision on the full thickness of the dorsal skin. 0.2 ml of vehicle or test agent was applied to the wound site and the wound was closed with four (4) silk 3-0 sutures at intervals of 1 cm. An Elizabethan collar was put on the rats approximately 5 to 6 hours post wounding for 5 to 7 days following recovery.

Carboxymethylcellulose gel (Carbopol) was used as the vehicle. Heparinase 3 was added to the vehicle as described above. Table 5 provides the treatment regimes used for each test group.

### Test System Groups:

| GROUP | MODEL | DOSE APPLICATION |
|---|---|---|
| 1 | N | L (vehicle); R (vehicle) |
| 2 | I | L (vehicle); R (vehicle) |
| 3 | N* | L (vehicle); R (Hep) |
| 4 | I | L (vehicle); R (Hep) |
| 5 | I | L (vehicle)*; R (Hep) |
| 6 | I | L (vehicle)**; R (Hep)** |

| | | |
|---|---|---|
| N, Normal model; I, Impaired model (30 mg/kg methylprednisolone, Depo-Medrol®); L, Left side wound; R, Right side wound *, Three applications (Day 0, Day 1 and Day 2) **, Seven applications (Day 0 through Day 6) | | |

Wound healing was evaluated on the basis of coaptation of the lips of the wound and on the physical aspects of the wound. For coaptation, 3 points/section was given for coapted lips, 2 points/section for coaptation less than or equal to 2 mm, and 1 point/section for coaption greater than or equal to 2 mm. For scoring the physical wound aspects, 5 points/section was given for apparent healing and/or disappearance of scab, 4 points/section for dry scab, 3 points/section for fresh scab, 2 point/section for humid wound and 1 point/section for fresh wound. Wound evaluation was noted everyday from day 1 to the day of sacrifice.

Healing was further evaluated on the basis of wound tensile strength. After sacrifice, skin sections containing the wound site were removed from the test animals. Wound tensile strength was measured using a 55 MN Mini Merlin^{™} tensometer.

A single intramuscular injection of methylprednisolone (30 mg/kg), two days prior to wounding, resulted in a significant reduction (59%) of the wound healing processes as measured by the wound tensile strength of skin sections of the impaired group (Group 2: left side: 0.735 ± 0.351 g/mm², right side: 0.919 ± 0.368 g/mm²) compared to the normal group (Group 1: left side: 2.007 ± 0.888 g/mm² right side: 1.989 ± 0.562 g/mm²) (p = 0.0001) as shwon in Figure 16.

In the normal rat model, a single application of heparinase 3 on Day 0 (Group 3: right side: 1.968 ± 0.748 g/mm²) did not result in a significant improvement of the mean wound tensile strength measurement compared to a single dose application of vehicle (Group 3: left side: 1.826 ± 0.804 g/mm²). In the impaired rat model, treated with methylpredinisolone, a single application of vehicle on Day 0 (Group 4: left side: 0.774 ± 0.265 g/mm²) showed a wound tensile strength measurement of 40% relative to the normal rats (1.941 ± 0.752 g/mm², mean of all normal wound tensile strength measurements: left and right sides of Group 1 and left side of Group 3). A single application of heparinase 3 on Day 0 (Group 4: right side: 1.253 ± 0.623 g/mm²) showed a wound tensile strength measurement of 65% relative to the normal rats.

In the impaired rat model, three applications of vehicle on consecutive days (Group 5: left side: 0.682 ± 0.301 g/mm²) resulted in a mean wound tensile strength measurement of 35% relative to the normal rats compared to 68% for three applications of heparinase 3(Group 5; right side: 1.322 ± 0.543 g/mm²). In the impaired rat model, seven applications of vehicle (Group 6: left side: 0.850 ± 0.81 2) resulted in a wound tensile strength measurement of 44% relative to the normal rats compared to 62% for seven applications of heparinase 3 (Group 6: right side: 1.206 ± 0.655 g/mm²).

### Example 16: Comparison of Heparinase 3 and dose-response of purified Heparinase 3 in wound healing in normal and glucocortoid-induced rat models.

The study described in Example 15 was repeated using different lots of heparinase 3 enzyme (lot hep3.00123) at concentrations of 0.02, 0.2, or 2.0 IU/wound. In addition, PBS was used as the vehicle in place of the carboxymethylcellulose gel vehicle used in Example 15. The treatment provided to each animal was as follows.

| GROUP | MODEL | DOSE APPLICATION |
|---|---|---|
| 1 | N | L (PBS); R (PBS) |
| 2 | I | L (PBS); R (PBS) |
| 3 | I | L (PBS); R (Hep) |
| 4 | I | L (PBS); R (Hep-P) |
| 5 | I | L (PBS); R (Hep-P) |
| 6 | I | L (PBS); R (Hep-P) |

N, Normal model; I, Impaired model (30 mg/kg methylprednisolone, Depo-Medrol®); L, Left side wound; R, Right side wound; WTS, Wounded Tensile Strength Analysis; Hep, Heparinase 3; Hep-P, Heparinase 3 - Purified.

A single injection of methylprednisolone (30 mg/kg), two days prior to wounding, resulted in a large reduction (46%) of the wound healing processes as measured by the mean wound tensile strength of skin sections of the impaired group (Group 2, left side: 0.70 ± 0.09, ± 0.28 g/mm², right side: 0.99 ± 0.09, ± 0.28 (mean ± SE, ± SD)) compared to the normal group (Group 1, left side: 1.52 ± 0.12, ± 0.57 g/mm², right side: 1.59 ± 0.13, ± 0.64 g/mm²), as shown in Figure 17, and Table 6.

In the impaired rat model, three applications of vehicle (Group 3, left side: 0.71 ± 0.07, ± 0.32 g/mm²) showed a mean wound tensile strength measurement representing an impairment of 54% relative to the normal rats (mean of all wound tensile strength measurements: left and right sides of Group 1: 1.55 ± 0.09, ± 0.60 g/mm²). Three applications of heparinase (Heparinase 3 lot HEPIII RH-67) showed a mean wound tensile strength measurement representing an impairment of 47% relative to the normal rats. Application of heparinase provided an impairment reversal effect of 7%, as shown by Figure 17 and Table 6.

In the impaired rat model, three applications of vehicle (Group 4, left side: 0.89 ± 0.09, ± 0.42 g/mm²) showed a mean wound tensile strength measurement representing an impairment of 43% relative to the normal rats. Three applications of Hep-P (Heparinase 3 lot HEPIII.001) Dose 1 (0.02 IU/200 µL) showed a wound tensile strength measurement representing an impairment of 35% relative to the normal rats. Application of Hep-P Dose 1 provided an impairment reversal effect of 8%, as shown by Figure 17 and Table 6.

In the impaired rat model, three applications of vehicle (Group 5, left side: 0.74 ± 0.04, ± 0.17 g/mm²) showed a mean wound tensile strength measurement representing an impairment of 53% relative to the normal rats. Three applications of Hep-P (Heparinase 3 lot HEPIII.001) Dose 2 (0.20 IU/200 µL) showed a wound tensile strength measurement representing an impairment of 26% relative to the normal rats. Application of Hep-P Dose 2 provided an impairment reversal effect of 27%, as shown by Figure 17 and Table 6.

In the Impaired rat model, three applications of vehicle (Group 6, left side: 0.72 ± 0.10, ± 0.30 g/mm²) showed a mean wound tensile strength measurement representing an impairment of 54% relative to the normal rats. Three applications of Hep-P (Heparinase 3 lot HEPIII.001) Dose 3 (2.00 IU/200 µL) showed a wound tensile strength measurement representing an impairment of 4% relative to the normal rats. Application of Hep-P Dose 3 provided an impairment reversal effect of 50%, as shown by Figure 17 and Table 6.

**Table 6: WOUND TENSILE STRENGTH (g/mm²)**

| | **GROUP 1** | **GROUP 2** | **GROUP 3** | **GROUP 4** | **GROUP 5** | **GROUP 6** |
|---|---|---|---|---|---|---|
| | L/R | L/R | L/R | L/R | L/R | L/R |
| AVG | 1.521 | 0.698 | 0.707 | 0.893 | 0.736 | 0.720 |
| | 1.585 | 0.987 | 0.819 | 1.016 | 1.147 | 1.492 |
| STD | 0.565 | 0.282 | 0.315 | 0.423 | 0.172 | 0.301 |
| | 0.638 | 0.282 | 0.498 | 0.467 | 0.562 | 0.815 |
| SE | 0.115 | 0.085 | 0.072 | 0.090 | 0.039 | 0.095 |
| | 0.130 | 0.085 | 0.114 | 0.101 | 0.129 | 0.258 |

## Claims

1. Use of a pharmaceutical composition in the manufacture of a medicament for enhancing wound healing by release of heparin binding growth factors and molecules from the extracellular matrix, removal of chondroitin sulfate from cell surface receptors and/or removal of the heparan sulfate component of their growth factor receptor complex, wherein the composition comprises a bacterial glycosaminoglycan degrading enzyme selected from the group consisting of heparinase 1 from *Flavobacterium heparinum,* heparinase 2 from *Flavobacterium heparinum,* heparinase 3 from *Flavobacterium heparinum,* chondroitinase AC from *Flavobacterium heparinum,* and chondroitinase B from *Flavobacterium heparinum,* heparinase from *Bacteroides* strains, heparinase from *Flavobacterium* Hp206, heparinase from *Cytophagia* species, chondroitin sulfate degrading enzymes from *Bacteroides* species, chondroitin sulfate degrading enzymes from *Proteus vulgaris,* chondroitin sulfate degrading enzymes from *Microcossus,* chondroitin sulfate degrading enzymes from *Vibrio* species, chondroitin sulfate degrading enzymes from *Arthrobacter aurescens,* these enzymes expressed from recombinant nucleotide sequences expressed in bacteria, and combinations thereof, in combination with a pharmaceutically acceptable carrier for localized administration.

2. The use of Claim 1 wherein the carrier is a pharmaceutically acceptable carrier for administration topically.

3. The use of Claim 2 wherein the carrier is an ointment, a polymeric film, a gel, a microparticulate, a microcapsule, a liposome, a proteosome, a liposphere, an implant, a transdermal patch, or a bandage.

4. The use of Claim 3 wherein the enzyme is incorporated into a polymeric matrix.

5. A delivery vehicle for delivery of, and comprising, enzyme in combination with the carrier in a dosage effective to enhance wound healing by release of heparin binding growth factors and molecules from the extracellular matrix, removal of chondroitin sulfate from cell surface receptors and/or removal of the heparan sulfate component of their growth factor receptor complex, wherein the enzyme is
a bacterial glycosaminoglycan degrading enzyme selected from the group consisting of heparinase 1 from *Flavobacterium heparinum,* heparinase 2 from *Flavobacterium heparinum,* heparinase 3 from *Flavobacterium heparinum,* chondroitinase AC from *Flavobacterium heparinum,* and chondroitinase B from *Flavobacterium heparinum,* heparinase from *Bacteroides* strains, heparinase from *Flavobacterium* Hp206, heparinase from *Cytophagia* species, chondroitin sulfate degrading enzymes from *Bacteroides* species, chondroitin sulfate degrading enzymes from *Proteus vulgaris,* chondroitin sulfate degrading enzymes from *Microcossus,* chondroitin sulfate degrading enzymes from *Vibrio* species, chondroitin sulfate degrading enzymes from. *Arthrobacter aurescens,* these enzymes expressed from recombinant nucleotide sequences expressed in bacteria, and combinations thereof, in combination with a pharmaceutically acceptable carrier wherein, if the enzyme is heparinase 1, 2 or 3 from *Flavobacterium heparinum,* then the composition does not comprise an effective amount of heparinase in a pharmaceutically acceptable carrier to inhibit angiogenesis at a selected site in a non-heparinized patient.

6. The delivery vehicle of Claim 5 wherein the delivery vehicle is a catheter or endoscope.

7. The use of Claim 1 wherein the glycosaminoglycan degrading enzyme is a fusion protein.

8. The use of Claim 1 further comprising a targeting molecule which binds specifically to a targeted cell and which is bound to the glycosaminoglycan degrading enzyme, wherein the targeting molecule is selected from proteins, polysaccharides, nucleic acids, or lipids.

9. The use of Claim 8 wherein the targeting molecule is selected from hormones, antibodies, integrins, or extracellular matrix binding molecules binding to cell surface molecules.

10. The use of Claim 1 wherein the enzyme is expressed from a recombinant nucleotide sequence in an organism is which it does not naturally occur and the enzyme is processed differently than in the organism in which it does not naturally occur.

11. The use of Claim 1 wherein the composition enhances wound healing of vasculature by preventing restenosis.

12. The use of a delivery vehicle according to Claim 5 or 6 in the manufacture of a device for enhancing wound healing by release of heparing binding growth factors and molecules from the extracellular matrix, removal of chondroitin sulfate from cell surface receptors and/or removal of the heparan sulfate component of their growth factor receptor complex.

13. A composition as defined in any one of Claims 1 to 4 or 7 to 10 wherein, if the enzyme is heparinase 1, 2 or 3 from *Flavobacterium heparinum*, then the composition does not comprise an effective amount of heparinase in a pharmaceutically acceptable carrier to inhibit angiogenesis at a selected site in a non-heparinized patient.

14. A composition as defined in Claim 13 wherein the carrier is a polymeric film, a microparticulate, a microcapsule, a protesome, a liposome, a transdermal patch or a bandage.

## Patentansprüche

1. Verwendung einer pharmazeutischen Zusammensetzung bei der Herstellung eines Medikaments zur Verbesserung der Wundheilung über die Freisetzung von heparinbindenden Wachstumsfaktoren und von Molekülen der extrazellulären Matrix, die Entfernung von Chondroitinsulfat von Zelloberflächenrezeptoren und/oder die Entfernung der Heparansulfatkomponente ihres Wachstumsfaktorrezeptor-Komplexes, wobei die Zusammensetzung umfasst ein bakterielles Glycosaminoglycan-abbauendes Enzym, das aus der Gruppe ausgewählt ist, die besteht aus der Heparinase 1 von *Flavobacterium heparinum,* der Heparinase 2 von *Flavobacterium heparinum,* der Heparinase 3 von *Flavobacterium heparinum,* der Chondroitinase AC von *Flavobacterium heparinum* und der Chondroitinase B von *Flavobacterium heparinum,* Heparinase von *Bacteroides*-Stämmen, Heparinase von *Flavobacterium* Hp206, Heparinase von *Cytophagia*-Species*,* Chondroitinsulfat-abbauenden Enzymen von *Proteus vulgaris,* Chondroitinsulfat-abbauenden Enzymen von *Microcossus,* Chondroitinsulfat-abbauenden Enzymen von *Vibrio*-Species, Chondroitinsulfat-abbauenden Enzymen von *Arthrobacter aurescens,* wobei diese Enzyme von rekombinanten, in Bakterien exprimierten Nucleotidsequenzen exprimiert werden, und Kombinationen von diesen, in Kombination mit einem pharmazeutisch annehmbaren Träger für eine lokalisierte Verabreichung.

2. Verwendung nach Anspruch 1, wobei der Träger ein pharmazeutisch annehmbarer Träger für eine topische Verabreichung ist.

3. Verwendung nach Anspruch 2, wobei der Träger eine Salbe, eine Polymerfolie, ein Gel, ein Mikropartikel, eine Mikrokapsel, ein Liposom, ein Proteosom, ein Liposphere, ein Implantat, ein transdermales Pflaster oder eine Bandage ist.

4. Verwendung nach Anspruch 3, wobei das Enzym in eine polymere Matrix inkorporiert ist.

5. Zuführungsvehikel für die Zuführung von einem, und umfassend ein, Enzym in Kombination mit dem Träger in einer Dosierung, die wirksam ist, die Wundheilung über die Freisetzung von heparinbindenden Wachstumsfaktoren und von Molekülen der extrazellulären Matrix, die Entfernung von Chondroitinsulfat von Zelloberflächenrezeptoren und/oder die Entfernung der Heparansulfatkomponente ihres Wachstumsfaktorrezeptor-Komplexes zu verbessern, wobei das Enzym ein bakterielles Glycosaminoglycan-abbauendes Enzym ist, das aus der Gruppe ausgewählt ist, die besteht aus der Heparinase 1 von *Flavobacterium heparinum,* der Heparinase 2 von *Flavobacterium heparinum,* der Heparinase 3 von *Flavobacterium heparinum,* der Chondroitinase AC von *Flavobacterium heparinum* und der Chondroitinase B von *Flavobacterium heparinum,* Heparinase von *Bacteroides*-Stämmen, Heparinase von *Flavobacterium* Hp206, Heparinase von *Cytophagia*-Species, Chondroitinsulfat-abbauenden Enzymen von *Proteus vulgaris,* Chondroitinsulfat-abbauenden Enzymen von *Microcossus,* Chondroitinsulfat-abbauenden Enzymen von *Vibrio*-Species, Chondroitinsulfat-abbauenden Enzymen von *Arthrobacter aurescens,* wobei diese Enzyme von rekombinanten, in Bakterien exprimierten Nucleotidsequenzen exprimiert werden, und Kombinationen von diesen, in Kombination mit einem pharmazeutisch annehmbaren Träger, wobei, wenn das Enzym die Heparinase 1, 2 oder 3 von *Flavobacterium heparinum* ist, die Zusammensetzung dann nicht eine zur Hemmung der Angiogenese an einer ausgewählten Stelle in einem nicht-heparinisierten Patienten wirksame Menge einer Heparinase in einem pharmazeutisch annehmbaren Träger umfasst.

6. Zuführungssystem nach Anspruch 5, wobei das Zuführungsvehikel ein Katheter oder ein Endoskop ist.

7. Verwendung nach Anspruch 1, wobei das Glycosaminogiycan-abbauende Enzym ein Fusionsprotein ist.

8. Verwendung nach Anspruch 1, ferner umfassend ein auf eine Zielstruktur abzielendes Molekül, das spezifisch an eine Zielzelle bindet und das an das Glycosaminoglycanabbauende Enzym gebunden ist, wobei das auf die Zielstruktur abzielende Molekül aus Proteinen, Polysacchariden, Nucleinsäuren oder Lipiden ausgewählt ist.

9. Verwendung nach Anspruch 8, wobei das auf eine Zielstruktur abzielende Molekül aus Hormonen, Antikörpern, Integrinen oder Bindungsmolekülen der extrazellulären Matrix, die an Zelloberflächenmoleküle binden, ausgewählt ist.

10. Verwendung nach Anspruch 1, wobei das Enzym von einer rekombinanten Nucleotidsequenz in einem Organismus exprimiert wird, in dem es natürlicherweise nicht vorkommt, und das Enzym anders prozessiert wird als in dem Organismus, in dem es natürlicherweise nicht vorkommt.

11. Verwendung nach Anspruch 1, wobei die Zusammensetzung die Wundheilung des Gefäßsystems über die Verhinderung einer Restenose verbessert.

12. Verwendung eines Zuführungsvehikels gemäß Anspruch 5 oder 6 bei der Herstellung einer Vorrichtung zur Verbesserung der Wundheilung über die Freisetzung von heparinbindenden Wachstumsfaktoren und von Molekülen der extrazellulären Matrix, die Entfernung von Chondroitinsulfat von Zelloberflächenrezeptoren und/oder die Entfernung der Heparansulfatkomponente ihres Wachstumsfaktorrezeptor-Komplexes.

13. Zusammensetzung, wie sie in einem beliebigen der Ansprüche 1 bis 4 oder 7 bis 10 definiert wird, wobei, wenn das Enzym die Heparinase 1, 2 oder 3 von *Flavobacterium heparinum* ist, die Zusammensetzung dann nicht eine zur Hemmung der Angiogenese an einer ausgewählten Stelle in einem nicht-heparinisierten Patienten wirksame Menge einer Heparinase in einem pharmazeutisch annehmbaren Träger umfasst.

14. Zusammensetzung, wie sie im Anspruch 13 definiert wird, wobei der Träger eine Polymerfolie, ein Mikropartikel, eine Mikrokapsel, ein Proteosom, ein Liposom, ein transdermales Pflaster oder eine Bandage ist.

## Revendications

1. Utilisation d'une composition pharmaceutique dans la fabrication d'un médicament pour améliorer la cicatrisation par libération de molécules et de facteurs de croissance se liant à l'héparine par la matrice extracellulaire, par séparation du chondroïtine sulfate des récepteurs de surface cellulaire et/ou séparation du composant héparane sulfate de leur complexe avec un récepteur à facteur de croissance, dans laquelle la composition comprend une enzyme bactérienne de dégradation des glycosaminoglycanes choisie dans le groupe constitué par l'héparinase 1 de *Flavobacterium heparinum,* l'héparinase 2 de *Flavobacterium heparinum,* l'héparinase 3 de *Flavobacterium heparinum,* la chondroïtinase AC de *Flavobacterium heparinum* et la chondroïtinase B de *Flavobacterium heparinum,* l'héparinase de souches de *Bacteroides*, l'héparinase de *Flavobacterium* Hp206, l'héparinase de l'espèce *Cytophagia,* les enzymes de dégradation du chondroïtine sulfate provenant de l'espèce *Bacteroides,* les enzymes de dégradation du chondroïtine sulfate provenant de *Proteus vulgaris,* les enzymes de dégradation du chondroïtine sulfate provenant de *Microcossus,* les enzymes de dégradation du chondroïtine sulfate provenant de l'espèce *Vibrio,* les enzymes de dégradation du chondroïtine sulfate provenant d'*Arthrobacter aurescens,* ces enzymes exprimées à partir de séquences de nucléotides recombinantes exprimées dans des bactéries, et leurs combinaisons, en combinaison avec un support pharmaceutiquement acceptable pour une administration localisée.

2. Utilisation selon la revendication 1, dans laquelle le support est un support pharmaceutiquement acceptable pour une administration topique.

3. Utilisation selon la revendication 2, dans laquelle le support est une pommade, un film polymère, un gel, une matière microparticulaire, une microcapsule, un liposome, un protéosome, une liposphère, un implant, un timbre transdermique ou un bandage.

4. Utilisation selon la revendication 3, dans laquelle l'enzyme est incorporée dans une matrice polymère.

5. Véhicule de délivrance pour délivrance de, et comprenant, une enzyme en combinaison avec le support en un dosage efficace pour améliorer la cicatrisation par libération de molécules et de facteurs de croissance se liant à l'héparine par la matrice extracellulaire, par séparation du chondroïtine sulfate des récepteurs de surface cellulaire et/ou séparation du composant héparane sulfate de leur complexe avec un récepteur à facteur de croissance, dans lequel l'enzyme est une enzyme bactérienne de dégradation des glycosaminoglycanes choisie dans le groupe constitué par l'héparinase 1 de *Flavobacterium heparinum,* l'héparinase 2 de *Flavobacterium heparinum,* l'héparinase 3 de *Flavobacterium heparinum,* la chondroïtinase AC de *Flavobacterium heparinum* et la chondroïtinase B de *Flavobacterium heparinum,* l'héparinase de souches de *Bacteroides*, l'héparinase de *Flavobacterium* Hp206, l'héparinase de l'espèce *Cytophagia,* les enzymes de dégradation du chondroïtine sulfate provenant de l'espèce *Bacteroides,* les enzymes de dégradation du chondroïtine sulfate provenant de *Proteus vulgaris,* les enzymes de dégradation du chondroïtine sulfate provenant de *Microcossus,* les enzymes de dégradation du chondroïtine sulfate provenant de l'espèce *vibrio,* les enzymes de dégradation du chondroïtine sulfate provenant d'*Arthrobacter aurescens,* ces enzymes exprimées à partir de séquences de nucléotides recombinantes exprimées dans des bactéries, et leurs combinaisons, en combinaison avec un support pharmaceutiquement acceptable dans lequel, si l'enzyme est de l'héparinase 1, 2 ou 3 de *Flavobacterium heparinum,* alors la composition ne comprend pas une quantité efficace d'héparinase dans un support pharmaceutiquement acceptable pour inhiber l'angiogenèse au niveau d'un site choisi chez un malade non héparinisé.

6. Véhicule de délivrance selon la revendication 5, le véhicule de délivrance étant un cathéter ou un endoscope.

7. Utilisation selon la revendication 1, dans laquelle l'enzyme de dégradation des glycosaminoglycanes est une protéine de fusion.

8. Utilisation selon la revendication 1, comprenant en plus une molécule de ciblage qui se lie de manière spécifique à une cellule ciblée et qui est liée à l'enzyme de dégradation des glycosaminoglycanes, dans laquelle la molécule de ciblage est choisie parmi les protéines, les polysaccharides, les acides nucléiques ou les lipides.

9. Utilisation selon la revendication 8, dans laquelle la molécule de ciblage est choisie parmi les hormones, les anticorps, les intégrines, ou des molécules de liaison à la matrice extracellulaire qui se lient à des molécules de la surface cellulaire.

10. Utilisation selon la revendication 1, dans laquelle l'enzyme est exprimée à partir d'une séquence de nucléotides recombinante dans un organisme dans lequel elle n'apparaît pas naturellement et l'enzyme est traitée autrement que dans l'organisme dans lequel elle n'apparaît pas naturellement.

11. Utilisation selon la revendication 1, dans laquelle la composition améliore la cicatrisation du système vasculaire en prévenant la resténose.

12. Utilisation d'un véhicule de délivrance selon la revendication 5 ou 6 dans la fabrication d'un dispositif pour améliorer la cicatrisation par libération de molécules et de facteurs de croissance se liant à l'héparine par la matrice extracellulaire, par séparation du chondroïtine sulfate des récepteurs de surface cellulaire et/ou séparation du composant héparane sulfate de leur complexe avec un récepteur à facteur de croissance.

13. Composition telle que définie dans l'une quelconque des revendications 1 à 4 ou 7 à 10 dans laquelle, si l'enzyme est de l'héparinase 1, 2 ou 3 de *Flavobacterium heparinum*, alors la composition ne comprend pas une quantité efficace d'héparinase dans un support pharmaceutiquement acceptable pour inhiber l'angiogenèse au niveau d'un site choisi chez un malade non héparinisé.

14. Composition talle que définie dans la revendication 13, dans laquelle le support est un film polymère, une matière microparticulaire, une microcapsule, un protéosome, un liposome, un timbre transdermique ou un bandage.
